(19) **Europäisches Patentamt European Patent Office Office européen des brevets**

(11) **EP 4 775 223 A1**

(12) **EUROPEAN PATENT APPLICATION**
published in accordance with Art. 153(4) EPC

(43) Date of publication:
**15.07.2026   Bulletin 2026/29**

(21) Application number: **24862087.4**

(22) Date of filing: **06.09.2024**

(51) International Patent Classification (IPC):
**A61K 39/395** (2006.01)    **A61P 17/06** (2006.01)
**C07K 16/24** (2006.01)    **A61P 37/02** (2006.01)

(52) Cooperative Patent Classification (CPC):
**A61K 39/395; A61P 17/06; A61P 37/02; C07K 16/24**

(86) International application number:
**PCT/CN2024/117492**

(87) International publication number:
**WO 2025/051249 (13.03.2025 Gazette 2025/11)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:   **07.09.2023   CN 202311148653**
     **04.09.2024   CN 202411232681**

(71) Applicant: **Innovent Biologics (Suzhou) Co., Ltd. Suzhou, Jiangsu 215123 (CN)**

(72) Inventors:
• **CAI, Chenghang**
  **Suzhou, Jiangsu 215123 (CN)**
• **QIAN, Lei**
  **Suzhou, Jiangsu 215123 (CN)**

(74) Representative: **Sagittarius IP Marlow International Parkway Marlow SL7 1YL (GB)**

Remarks:
The complete document including Reference Table(s) and the Sequence Listing(s) can be downloaded from the EPO website

(54) **METHOD FOR TREATING MODERATE AND SEVERE PSORIASIS USING RECOMBINANT ANTI-IL-23P19 ANTIBODY**

(57)    The present disclosure relates to the field of disease treatment. More specifically, the present disclosure relates to use of a recombinant anti-IL-23p19 antibody in treating moderate to severe psoriasis, and also relates to a method for treating moderate to severe psoriasis in a subject, comprising administering to the subject an effective amount of an anti- IL-23 p19 antibody.

**EP 4 775 223 A1**

**Description**

**FIELD OF THE INVENTION**

The present disclosure relates to the field of disease treatment. More specifically, the present disclosure relates to use of a recombinant anti-IL-23p19 antibody in treating moderate to severe psoriasis, and also relates to a method for treating moderate to severe psoriasis in a subject, comprising administering to the subject an effective amount of an anti-IL-23 p19 antibody.

**Background of the Invention**

IL-23 is a heterodimeric cytokine of IL-12 family members, consisting of a p40 subunit and a p19 subunit. IL-23 activates signaling pathways by binding to the IL-23 receptor (IL-23R) and the $\beta1$ subunit of the IL-12 receptor (IL-12R$\beta$1). Chronic inflammation resulting from dysregulation of IL-23/Th17/IL-17 responses is the pathophysiological basis for a number of autoimmune diseases including psoriasis, ulcerative colitis, Crohn's disease, rheumatoid arthritis, multiple sclerosis and asthma.

Psoriasis is a chronic, recurrent, inflammatory, and systemic disease that is genetically and environmentally mediated and immune- mediated, and can occur at all ages without gender differences. The typical clinical manifestations are scaly erythema or plaques, localized or widespread, non-infectious, difficult to treat, and often lifelong. Psoriasis can be divided into vulgaris psoriasis (including guttate psoriasis and plaque psoriasis), pustular psoriasis, erythrodermic psoriasis, and arthropathic psoriasis. Currently, about 2% of the world's population has psoriasis, 80-90% of patients have plaque psoriasis, and nearly one third have moderate to severe psoriasis. The prevalence of psoriasis is significantly different around the world. According to a survey in 6 cities in China in 2008, it is estimated that the number of psoriasis patients in China is more than 6 million.

At present, the main systemic drug therapies for psoriasis in China include methotrexate (MTX), cyclosporine A, retinoids, and biological agents. In recent years, monoclonal antibody biological agents against cellular inflammatory factors have been successively used in the treatment of severe psoriasis accompanied by significant joint symptoms due to poor treatment with traditional systemic drugs, which seriously affects the quality of life, including, for example, tumor necrosis factor (TNF-$\alpha$) antagonists (etanercept, infliximab, and adalimumab), IL-12/23 antagonists (ustekinumab), and IL-17A antagonists (secukinumab). Among them, humanized IgG1 monoclonal antibodies that bind to the IL-23p19 subunit Guselkumab (developed by Johnson & Johnson), Risankizumab (developed by AbbVie), and Tildrakizumab (developed by Merck Sharp & dohme) in recent years have shown significant efficacy in the treatment of plaque psoriasis and have been used in many countries for the treatment of moderate to severe plaque psoriasis. At present, the utilization rate of biologics for psoriasis patients in China is still very low, and there is still a huge demand for effective, safe, and low-cost domestic biologics.

Guselkumab, an IL-23p19 antagonist, was approved in China in December 2019 for the treatment of adults patients with moderate to severe plaque psoriasis who are suitable for systemic therapy. The stable treatment with Guselkumab is administered once every 8 weeks or every 4 weeks. After withdrawal, its efficacy and the proportion of patients maintaining efficacy continue to decline over time. Therefore, there is still a need in the art to seek products that can maintain efficacy for a longer period of time after drug withdrawal, so as to reduce the burden on patients and improve their quality of life.

The present application meets these urgent needs to a certain extent.

**SUMMARY OF THE INVENTION**

The inventors of the present application have surprisingly found that since the anti-IL-23p19 antibody of the present disclosure has a long half-life, long-interval administration is allowed, and the antibody can be administered once every 12 weeks after stable treatment. The inventors of the present application have also found that due to the long half-life of the antibody of the present disclosure, the efficacy can be maintained for a longer time after drug withdrawal, and more patients maintain efficacy within the same time after drug withdrawal.

It can be seen that by administering the anti-IL-23p19 antibody of the present disclosure, particularly according to the dosing regimen of the present disclosure, an encouraging efficacy is obtained in patients with moderate to severe psoriasis, the skin lesions of the patients are significantly improved and the overall safety and tolerability are improved, a longer dosing interval is provided, the efficacy is maintained longer after drug withdrawal, thereby reducing the burden on the patients, and/or the quality of life is improved.

The inventors of the present application also surprisingly found that administration of the anti-IL-23p19 antibody according to the dosing regimen of the present disclosure can produce favorable efficacy in treating moderate to severe psoriasis, and the safety is acceptable and controllable.

Thus,

In a first aspect, the present disclosure provides a method for treating moderate to severe psoriasis in a subject, which comprises administering to a subject in need thereof an effective amount of an anti-IL-23P19 antibody.

In a second aspect, the present disclosure provides an anti-IL-23P19 antibody for use in treating moderate to severe psoriasis.

In a third aspect, the present disclosure provides use of the anti-IL-23P19 antibody disclosed herein in the manufacture of a medicament for treating moderate to severe psoriasis.

In a fourth aspect, the present disclosure provides a pharmaceutical composition comprising the anti-IL-23P19 antibody of the present disclosure and one or more pharmaceutically acceptable excipients.

In a fifth aspect, the present disclosure provides a single pharmaceutical dosage unit comprising the anti-IL23p19 antibody of the present disclosure.

In a sixth aspect, the present disclosure relates to a pharmaceutical kit comprising the anti-IL23p19 antibody of the present disclosure or the single pharmaceutical dosage unit of the fifth aspect.

In a seventh aspect, the present disclosure relates to use of the single pharmaceutical dosage unit according to the fifth aspect or the pharmaceutical kit according to the sixth aspect in the manufacture of a medicament for treating moderate to severe psoriasis.

It should be understood that the technical solutions obtained from any combination of any of the technical features described in the first to seventh aspects above are also included in the present disclosure.

Other embodiments of the present disclosure will be apparent from and elucidated with reference to the following detailed description.

## DETAILED DESCRIPTION OF THE INVENTION

Before describing the present disclosure in detail, it is to be understood that this disclosure is not limited to the particular methods and experimental conditions described herein, as such methods and conditions may vary. Additionally, the terminology used herein is for the purpose of describing particular embodiments only and is not intended to be limiting.

## DEFINITIONS

Unless defined otherwise, all technical and scientific terms used herein have the same meaning as commonly understood by one of ordinary skill in the art. For the purposes of the present disclosure, the following terms are defined below.

The term "about", when used in conjunction with a numerical value, is intended to encompass the numerical values in a range from a lower limit less than the specified numerical value by 5% to an upper limit greater than the specified numerical value by 5%.

The term "and/or", when used to connect two or more options, should be understood to refer to any one of the options or any two or more of the options.

As used herein, the term "comprise" or "include" is intended to mean that the described elements, integers or steps are included, but not to the exclusion of any other elements, integers or steps. As used herein, the term "comprise" or "include", unless otherwise specified, also encompasses the situation where the entirety consists of the described elements, integers or steps. For example, when referring to an antibody variable region "comprising" a particular sequence, it is also intended to encompass an antibody variable region consisting of that particular sequence.

The p19 subunit of IL-23 (also referred to herein as "IL-23p19" and "p19 subunit") is a polypeptide of 189 amino acids containing a leader sequence of 21 amino acids (Oppmann et al., Immunity 13:715 (2000), SEQ ID NO: 181) and comprising four packed alpha helices designated A, B, C and D, with an up-up-down-down topology. The four helices are connected by three polypeptide loops. The A-B and C-D loops are made relatively long because they connect parallel helices. The short B-C loop connects the antiparallel B and C helices. The p19 subunit of IL-23 is a member of the IL-6 family of helical cytokines. This cytokine family binds to its cognate receptors via 3 conserved epitopes (sites I, II, and III; Bravo and Heath (2000) EMBO J. 19:2399-2411). The p19 subunit interacts with three cytokine receptor subunits to form a competent signal transduction complex. When expressed in a cell, the p19 subunit first forms a complex with the p40 subunit, which is shared by the p19 subunit and IL-12. The p19p40 complex is secreted from cells as a heterodimeric protein and is referred to as IL-23. In one embodiment, the IL-23p19 of the present disclosure is derived from human (NCBI:AAG37232) or cynomolgus monkey (NCBI:AEY84629).

The term "anti-IL-23p19 antibody", "anti-IL-23p19", "recombinant anti-IL23p19 antibody", "IL-23p19 antibody", or "antibody that binds to IL-23p19" as used herein refers to an antibody that is capable of binding to a (human or cynomolgus monkey) IL-23p19 subunit or a fragment thereof with sufficient affinity such that the antibody can be used as a diagnostic and/or therapeutic agent in targeting (human or cynomolgus monkey) IL-23p19.

As used herein, the term "antibody" is used in the broadest sense, refers to a protein comprising an antigen-binding site, and encompasses natural and artificial antibodies of various structures, including but not limited to intact antibodies and antigen-binding fragments of antibodies.

The terms "whole antibody", "full-length antibody", "complete antibody" and "intact antibody" are used interchangeably herein to refer to a glycoprotein comprising at least two heavy (H) chains and two light (L) chains interconnected by disulfide bonds. Each heavy chain consists of a heavy chain variable region (abbreviated herein as VH) and a heavy chain constant region. The heavy chain constant region consists of 3 domains, CH1, CH2 and CH3. Each light chain consists of a light chain variable region (abbreviated herein as VL) and a light chain constant region. The light chain constant region consists of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs arranged from amino-terminus to carboxy-terminus in the following order: FR1, CDR1, FR2, CDR2, FR3, CDR3, FR4. The constant region is not directly involved in the binding of an antibody to an antigen, but exhibits various effector functions.

A "complementarity determining region" or "CDR region" or "CDR" is a region in an antibody variable domain that is highly variable in sequence and forms structurally defined loops ("hypervariable loops") and/or contains antigen-contacting residues ("antigen-contacting sites"). The CDRs are primarily responsible for binding to antigenic epitopes. The CDRs of the heavy and light chains are generally referred to as CDR1, CDR2, and CDR3, and are numbered sequentially from the N-terminus. The CDRs located in the heavy chain variable domain of an antibody are referred to as HCDR1, HCDR2 and HCDR3, and the CDRs located in the light chain variable domain of an antibody are referred to as LCDR1, LCDR2 and LCDR3.

In a given amino acid sequence of a light chain variable region or a heavy chain variable region, the exact amino acid sequence boundary of each CDR can be determined using any one or a combination of many well-known antibody CDR assignment scheme including, for example, Chothia based on the three-dimensional structure of antibodies and the topology of CDR loops (Chothia et al. (1989) Nature 342:877-883, Al-Lazikani et al., "Standard conformations for the canonical structures of immunoglobulins", Journal of Molecular Biology, 273, 927-948 (1997)), Kabat based on antibody sequence variability (Kabat et al., Sequences of Proteins of Immunological Interest, 4th edition, U.S. Department of Health and Human Services, National Institutes of Health (1987)), AbM (University of Bath), Contact (University College London), International ImMunoGeneTics database (IMGT) (on the World Wide Web imgt.cines.fr/), and North CDR definition based on the affinity propagation clustering using a large number of crystal structures.

Exemplary AbM rules are as follows:

| CDR | AbM scheme |
|-------|------------|
| LCDR1 | L24-L34 |
| LCDR2 | L50-L56 |
| LCDR3 | L89-L97 |
| HCDR1 | H26-H35B |
| HCDR2 | H50-H58 |
| HCDR3 | H95-H102 |

Unless otherwise stated, residue positions of an antibody variable region (including heavy chain variable region residues and light chain variable region residues) are numbered according to the Kabat numbering system (Kabat et al., Sequences of Proteins of Immunological Interest, 5th Ed. Public Health Service, National Institutes of Health, Bethesda, Md. (1991)).

In one embodiment, the boundaries of the CDRs of the antibody of the present disclosure are determined by AbM rules.

The term "antibody fragment" refers to a molecule other than an intact antibody that comprises a portion of an intact antibody and binds to the antigen to which the intact antibody binds. Examples of antibody fragments include, but are not limited to, Fv, Fab, Fab', Fab'-SH, F(ab')2; diabodies; linear antibodies; single-chain antibodies (e.g., scFv); single-domain antibodies; bivalent or bispecific antibodies or fragments thereof; camelid antibodies; and bispecific or multispecific antibodies formed from antibody fragments.

The term "psoriasis" as used herein is a non-communicable disease of unknown etiology for which the immune system is generally considered to play a role. Psoriasis has a genetic tendency and can be induced by external factors such as infection. Psoriasis includes, but is not limited to, psoriasis vulgaris (including guttate psoriasis and plaque psoriasis), pustular psoriasis, erythrodermic psoriasis, and arthropathic psoriasis (e.g., psoriatic arthritis). The psoriasis described herein may be any degree of psoriasis, e.g., mild, moderate, or severe psoriasis, wherein moderate and severe psoriasis ("moderate-to-severe psoriasis") are associated with a higher risk of complications, such as ischemic heart disease, stroke, hypertension, dyslipidemia, diabetes, and Crohn's disease. Clinical guidelines define moderate disease as more than 3% of the cumulative body surface area and severe disease as more than 10%. The actual enrollment of "moderate to severe psoriasis" in the population enrolled in the clinical trial was more than 10%, and the enrollment conditions of this study also followed this standard. Preferably, the psoriasis described herein is moderate to severe psoriasis, e.g., moderate to severe plaque psoriasis. Psoriasis often presents as one or more red, silvery, shiny patches on the scalp, elbows, knees, back, or buttocks. The skin around the eyebrows, armpits, navel, anus, and the junction of the buttocks and lower back may also be affected. It can also manifest as deformed, thickened, and sunken nails. Psoriasis can also be classified into, for example, scalp psoriasis, nail psoriasis, palmoplantar psoriasis, perineal region psoriasis, etc., depending on the site involved.

The term "Week 0" as used herein refers to the time point of the first administration of the IL-23p19 antibody. The term "Week 4" as used herein refers to the 4th week calculated from the time point of the first administration of the IL-23p19 antibody. The term "Week 8" as used herein refers to the 8th week calculated from the time point of the first administration of the IL-23p19 antibody. Other similar terms should also be understood accordingly.

"Treating" refers to slowing, interrupting, arresting, alleviating, stopping, reducing, or reversing the progression or severity of an existing symptom, disorder, condition, or disease.

The term "effective amount" refers to an amount or dosage of the formulation or antibody of the present disclosure that produces the desired effect in a treated patient after administration to the patient in a single or multiple doses. The effective amount can be readily determined by an attending physician as a person skilled in the art by considering a variety of factors as follows: species such as mammals; its size, age, and general health; the specific disease involved; the degree or severity of the disease; response in an individual patient; specific antibody administered; mode of administration; bioavailability characteristics of the administered formulation; selected administration regimen; and use of any concomitant therapy.

A "therapeutically effective amount" refers to an amount that, at the required dosage and for the required period of time, effectively achieves the desired therapeutic outcome. A therapeutically effective amount of the formulation, antibody or antibody fragment, or conjugate or composition thereof of the present disclosure may vary depending on a variety of factors such as the disease state, age, sex, and weight of the individual, and the ability of the antibody or antibody portion to elicit a desired response in the individual. The therapeutically effective amount is also such an amount that any toxic or detrimental effect of the formulation, antibody or antibody fragment, or conjugate or composition thereof is inferior to the therapeutically beneficial effect.

A "formulation" or "pharmaceutical composition" refers to a composition comprising at least one active ingredient and at least one inactive ingredient which is suitable for administration to animals, preferably mammals, including humans. A "liquid formulation" or "liquid composition" refers to a formulation in liquid form. The liquid composition of the present disclosure comprises (i) the antibody of the present disclosure; (ii) a buffer; and (iii) a vehicle. The components of the formulation of the present disclosure may be as shown in the embodiments relating to the liquid pharmaceutical composition above. The liquid formulation disclosed herein is preferably an injection.

A "pharmaceutically acceptable carrier" refers to an ingredient in a pharmaceutical formulation, other than the active ingredient, which is not toxic to the subject. Pharmaceutically acceptable carriers include, but are not limited to, buffers, excipients, stabilizers, or preservatives.

As used herein, "buffer" refers to a pH buffer. For example, the buffer is selected from histidine, glutamate, phosphate, acetate, citrate, and tris(hydroxymethyl)aminomethane.

As used herein, the term "vehicle" refers to a liquid used to dissolve or suspend an active ingredient and an inactive ingredient to form a liquid formulation. Vehicles that can be used in the present disclosure include, but are not limited to, water for injection, organic solvents for injection including, but not limited to, oil for injection, ethanol, propylene glycol, and the like, or combinations thereof.

As used herein, the term "single pharmaceutical dosage unit" refers to a single pharmaceutical dosage form comprising the antibody of the present disclosure to be administered to a subject at the time of administration, such as a vial, an ampoule, a pre-filled syringe or a pre-filled syringe for injection, which contains a solution or a lyophilized powder of the drug.

The term "static Physician Global Assessment (sPGA)" is used to record the physician's assessment of the subject's psoriasis status, including the following aspects: induration, scaling, and erythema. It is used to evaluate the psoriasis skin lesions of a subject at a given time point. All skin lesions were graded according to the following ranges of induration, erythema, and scaling. The sPGA score is obtained by dividing the total score of the 3 items by 3, as shown below.

Induration (I) (average of all skin lesions; measured using the National Psoriasis Foundation Reference card)

0 = No evidence of plaque elevation
1 = microplaque elevation, 0.25mm
2 = slight plaque elevation, 0.5 mm
3 = Moderate plaque elevation, 0.75 mm
4 = Marked plaque elevation, 1 mm
5 = Severe plaque elevation, $\geq$ 1.25 mm
Erythema (E) (average of all skin lesions)
0 = No evidence of erythema, hyperpigmentation may be present
1 = faint erythema
2 = slight red color
3 = Moderately red
4 = Bright Red
5 = Dark red to deep red
Scaling (S) (average of all skin lesions)
0 = No evidence of scaling
1 = Slight scaling; occasional scaling <5% of total skin lesions
2 = Mild; predominately fine scaling
3 = moderate; coarse scale predominates
4 = Marked; thick, non-recalcitrant scale predominates
5 = Severe; very thick and recalcitrant scales predominate

$$\text{sPGA Score} = (I+E+S)/3 \text{ (Total Average)}$$

sPGA (static Physician Global Assessment) is evaluated based on the total average score:
0 = clear, except for some residual discoloration
1 = minimal, most skin lesions are individually scored 1
2 = Mild, most skin lesions are individually scored 2
3 = moderate, most skin lesions are individually scored 3
4 = marked, most skin lesions are individually scored 4
5 = Severe, most skin lesions are individually scored 5
Note: The fraction should be rounded to the nearest whole number. If the total score is < 1.50, the score = 1; if the total score is $\geq$ 1.50, the score = 2.

sPGA-G uses the evaluation criteria of sPGA to conduct a local assessment of perineal psoriasis, with a score of 0-5.

The term "Palmoplantar Psoriasis Severity Index" (PPASI) is a scoring system specifically used to assess the severity of palmoplantar psoriasis (i.e., palmar and plantar psoriasis). If subjects have baseline palmar-plantar psoriasis, the physician should assess at the protocol-specified visit points. Palms of each hand and soles of each foot were assessed separately from erythema, induration and scaling and the area affected. Calculated as follows:

The scoring is based on the proportion of the area of the palms or soles affected by the skin lesions, as follows:
0 = Not involved

$$1 = <10\%$$

$$2 = 10\% \leq \text{range} < 30\%$$

$$3 = 30\% \leq \text{range} < 50\%$$

$$4 = 50\% \leq \text{range} < 70\%$$

$$5 = 70\% \leq \text{range} < 90\%$$

$$6 = 90\% \leq \text{range} \leq 100\%$$

Skin lesions were then evaluated for erythema, induration, and scaling, with a score of 0-4 for each count.

0 = Clean
1 = Mild
2 = Medium
3 = heavy
4 = very heavy

PPASI is a composite score that calculates the score of each palm and sole, which is obtained by multiplying the sum of the scores of erythema, induration, and scaling by the score of the affected area of the palms and soles multiplied by a weight coefficient (weight coefficient of 0.2 for each palm, and weight coefficient of 0.3 for each foot).
The PPASI is calculated as follows:

(Sum of scores of erythema + induration + scaling) × lesion involvement score × 0.2 (right palm) + (sum of scores of erythema + induration + scaling) × lesion involvement score × 0.2 (left palm) + (sum of scores of erythema + induration + scaling) × lesion involvement score × 0.3 (right sole) + (sum of scores of erythema + induration + scaling) × lesion involvement score × 0.3 (left sole)

The value ranges from 0 to 72.
The term "Psoriasis Area and Severity Index, PASI" is a systematic tool for assessing and grading the severity of psoriatic skin lesions and response to treatment. PASI scores range from 0 to 72. The percentage of body surface area affected can be linearly combined with the severity of erythema, induration, and scaling at the four body sites. This endpoint is based on percent reduction relative to the baseline, usually summarized as a binomial distribution based on achieving X% reduction (or PSAIx), where X can be 50, 75, 90, and 100. To calculate PASI, the areas of four major body parts were evaluated: head (h), trunk (t), upper extremities (u), and lower extremities (l), which account for 10%, 30%, 20% and 40% of the total body surface area respectively.
The areas affected by psoriasis in these four parts are expressed numerically:
0 = not involved;

$$1 = < 10\%;$$

$$2 = 10\% \text{ to } < 30\%;$$

$$3 = 30\% \text{ to } < 50\%;$$

$$4 = 50\% \text{ to } < 70\%;$$

$$5 = 70\% \text{ to } < 90\%;$$

$$6 = 90\% - 100\%$$

The erythema (E), induration (I), and scaling (S) of the skin lesion indicating the severity were evaluated using a scale of 0-4, where 0 represents no skin involvement at all, 1 is mild, 2 is moderate, 3 is severe, and 4 is very severe; the scores of the four sites were listed separately.
To aid area evaluation, note the following points:

a. Neck belongs to the head
b. Armpits and groins belong to trunk
c. Buttocks belong to the lower limbs PASI = 0.1 * (Eh + Ih + Sh) Ah + 0.3 * (Et + It + St) At + 0.2 * (Eu + Iu + Su) Au + 0.4 * (El + Il + Sl) Al.

The term "Dermatology Life Quality Index, DLQI" is a patient-administered, ten-question quality-of-life questionnaire covering 6 aspects: symptoms and feelings, daily activities, leisure, work and study, interpersonal relationships, and treatment. The DLQI is a one-week recall period. Response categories included not related (0), not at all (0), few (1), serious (2), very serious (3). The answer to question 7 was "Yes"/"No", "Yes" isscored 3 points.

The DLQI is to be filled out by the subject independently during the visits marked in the flow chart.

The DLQI was analyzed by the following 6 aspects:

| Dimension | Issue No. | Score |
|---|---|---|
| Symptoms and Feelings | Questions 1 and 2 | Score up to 6 |
| Daily Activities | Questions 3 and 4 | Score up to 6 |
| Leisure | Questions 5 and 6 | Score up to 6 |
| Work and study | Q7 | Score up to 3 |
| Interpersonal relationships | Questions 8 and 9 | Score up to 6 |
| Treatment | Q10 | Score up to 3 |

Score corresponding to the option

| Very Much | Many | A little | Not at all | Not related | Not answered |
|---|---|---|---|---|---|
| 3 | 2 | 1 | 0 | 0 | 0 |

For item 7, select "Yes", the score reaches the maximum of 3 points, select "No", and select the score in the table according to the options.

The DLQI total score was derived by summing each question score, with results ranging from 0 to 30. The higher the score, the worse the quality of life.

0-1 points = not affecting the subject's life,
2-5 points = small effect,
6-10 points = moderate impact,
11-20 points = very large impact,
21-30 points = having a great impact on the subject's life.

If a question answer is missing for a dimension, the dimension is considered missing. If 2 or more questions were not answered, the DLQI total score was considered missing. A change of 5 points from baseline was considered a clinically important difference.

The term "The Psoriasis Scalp Severity Index, PSSI" is a system for evaluating and grading scalp psoriatic scalp lesions and their response to treatment. The counting score of the PSSI ranges from 0 to 72 points. Disease severity was calculated as follows:

The scalp was evaluated according to the cumulative range (in terms of hairline perimeter) and scored as follows:

0 = Not involved

$$1 = <10\%$$

$$2 = 10\% \leq range < 30\%$$

$$3 = 30\% \leq range < 50\%$$

$$4 = 50\% \leq range < 70\%$$

$$5 = 70\% \leq range < 90\%$$

$$6 = 90\% \leq range \leq 100\%$$

The scalp lesions were then evaluated for erythema, induration, and scaling, with a score of 0-4 for each count.

0 = Clean
1 = Mild
2 = Medium
3 = heavy
4 = very heavy

$$PSSI = (Sum\ of\ Erythema,\ Induration\ and\ Scaling\ Score) \times (Scalp\ Cumulative\ Range\ Score)$$

The term "Nail Psoriasis Severity Index, NAPSI" is a tool used to assess the severity of nail lesions in patients with psoriasis. It scores each nail according to the characteristics of the lesion on the nail (e.g., pitting, discoloration, nail plate detachment, etc.), and then sums up to give a total score. If the subject has nail psoriasis, the physician should assess the nail psoriasis at the protocol-specified visit points. Two independent evaluations were performed separately on the fingers (5) of each hand and graded as follows:

Nail matrix assessment
0 = None
1 = 1 quadrant of the cumulative nail
2 = 2 quadrants of the cumulative nail
3 = 3 quadrants of the cumulative nail
4 = 4 quadrants of the cumulative nail
Nail bed evaluation
0 = None
1 = 1 quadrant of the cumulative nail
2 = 2 quadrants of the cumulative nail
3 = 3 quadrants of the cumulative nail
4 = 4 quadrants of the cumulative nail

The sum of the scores is in the range of 0-80 points. If one finger is not evaluated, the final score is the average of the remaining fingers plus the total number. If <50% of fingers are not evaluated, estimate using this method; if >50% of fingers are not evaluated, the total score is considered missing.

The term "Global Assessment of Disease Activity for PsA" is a tool used to assess psoriatic arthritis (PsA). It usually includes an assessment of indicators such as arthritic symptoms, joint swelling, pain, and dysfunction, to help doctors determine the severity of the disease and the effect of treatment.

Criteria for diagnosis and treatment of psoriatic arthritis

Patients with inflammatory arthropathy of the joints, spine, or tendon segments are evaluated, and a total score of 3 or more of the following five items is diagnostic of psoriatic arthritis:

1. Evidence of psoriasis (not recalculated)

- Current medical history of psoriatic skin lesions found by a dermatologist or rheumatologist, 2 points
- Personal history of psoriasis confirmed by the patient, dermatologist, rheumatologist, or other qualified medical personnel, 1 point
- Patient reports a family history of psoriasis in a first- or second-degree relative, 1 point

2. Physical examination reveals typical psoriatic nail changes, 1 point
3. Rheumatoid factor negative, 1 point
Any method other than gel-clot assay, preferably enzyme-linked immunosorbent assay or turbidimetry
4. Dactylitis (not recalculated)

- Current medical history of swelling of the whole finger (toe), 1 point
- History of dactylitis recorded by a rheumatologist/dermatologist, 1 point

5. Radiographic evidence of new bone formation near the joint end, 1 point
X-rays of hands and feet show poorly defined ossification of joint margins (osteophytes should be excluded ).

Methods of treatment

The inventors have determined the use and suitable optimized dosage regimen of the anti-IL-23p19 antibody of the present disclosure for the treatment of moderate to severe psoriasis through carefully designed clinical trials, taking into comprehensive consideration preclinical efficacy results, as well as pharmacokinetics, pharmacodynamics, immunogenicity, and toxicology in human subjects.

In a first aspect, the present disclosure relates to a method for treating moderate to severe psoriasis, comprising administering an effective amount of an anti-IL-23p19 antibody to a subject in need thereof.

In some embodiments, the present disclosure provides a method for treating moderate to severe psoriasis in a subject with an anti-IL-23p19 antibody, comprising administering to the subject an effective amount of the anti-IL-23p19 antibody.

For the first aspect, further embodiments thereof are as follows:

In some embodiments, the anti-IL23p19 antibody comprises a heavy chain variable region and a light chain variable region, wherein the CDR1 of the heavy chain variable region comprises or consists of the amino acid sequence of GYTFTSYLMH (SEQ ID NO: 1), the CDR2 of the heavy chain variable region comprises or consists of the amino acid sequence of YINPYNEGTN (SEQ ID NO: 2), and the CDR3 of the heavy chain variable region comprises or consists of the amino acid sequence of NWDLPY (SEQ ID NO: 3); the CDR1 of the light chain variable region of the antibody comprises or consists of the amino acid sequence of RASQSISDYLH (SEQ ID NO: 4), the CDR2 of the light chain variable region comprises or consists of the amino acid sequence of YASQSMS (SEQ ID NO: 5), and the CDR3 of the light chain variable region comprises or consists of the amino acid sequence of QQGHSFPFT (SEQ ID NO: 6). In some embodiments, the boundaries of the CDRs are determined by AbM rules.

In some embodiments, the recombinant anti-IL-23p19 antibody comprises a heavy chain variable region VH and a light chain variable region VL, wherein the heavy chain variable region comprises the sequence of SEQ ID NO: 7 or a sequence having at least 90%, 95%, 98% or 99% identity to SEQ ID NO: 7, and the light chain variable region comprises the sequence of SEQ ID NO: 8 or a sequence having at least 90%, 95%, 98% or 99% identity to SEQ ID NO: 8:

Sequence (SEQ ID NO: 7)

QVQLVQSGAEVKKPGASVKVSCKASGYTFTSYLMHWVRQAPGQGLEWMGYINPYN

EGTNYAQKFQGRVTMTRDTSISTAYMELSRLRSDDTAVYYCARNWDLPYWGQGTLVTVSS ;

Sequence (SEQ ID NO: 8)

DIQMTQSPSSLSASVGDRVTITCRASQSISDYLHWYQQKPGKAPKLLIKYASQSMSGVP

SRFSGSGSGSDFTLTISSLQPEDFATYYCQQGHSFPFTFGQGTKLEIK。

In some embodiments, the recombinant anti-IL-23p19 antibody is an IgG1 antibody, preferably comprising a heavy chain sequence of SEQ ID NO: 9 or a sequence having at least 90%, 95%, 98%, or 99% identity to SEQ ID NO: 9, and a light chain sequence of SEQ ID NO: 10 or a sequence having at least 90%, 95%, 98%, or 99% identity to SEQ ID NO: 10.

Sequence ( SEQ ID NO: 9 )

QVQLVQSGAEVKKPGASVKVSCKASGYTFTSYLMHWVRQAPGQGLEWMGYINPYNE

GTNYAQKFQGRVTMTRDTSISTAYMELSRLRSDDTAVYYCARNWDLPYWGQGTLVTVSSA

STKGPSVFPLAPSSKSTSGGTAALGCLVKDYFPEPVTVSWNSGALTSGVHTFPAVLQSSGLYS

LSSVVTVPSSSLGTQTYICNVNHKPSNTKVDKKVEPKSCDKTHTCPPCPAPELLGGPSVFLF

PPKPKDTLYITREPEVTCVVVDVSHEDPEVKFNWYVDGVEVHNAKTKPREEQYNSTYRVV

SVLTVLHQDWLNGKEYKCKVSNKALPAPIEKTISKAKGQPREPQVYTLPPSRDELTKNQVS

LTCLVKGFYPSDIAVEWESNGQPENNYKTTPPVLDSDGSFFLYSKLTVDKSRWQQGNVFSCS

VMHEALHNHYTQKSLSLSPG

Sequence ( SEQ ID NO: 10 )

DIQMTQSPSSLSASVGDRVTITCRASQSISDYLHWYQQKPGKAPKLLIKYASQSMSGVP

SRFSGSGSGSDFTLTISSLQPEDFATYYCQQGHSFPFTFGQGTKLEIKRTVAAPSVFIFPPSDEQ

LKSGTASVVCLLNNFYPREAKVQWKVDNALQSGNSQESVTEQDSKDSTYSLSSTLTLSKA

DYEKHKVYACEVTHQGLSSPVTKSFNRGEC

In some embodiments, the recombinant anti-IL-23p19 antibody comprises a heavy chain set forth in SEQ ID NO: 9 and a light chain set forth in SEQ ID NO: 10. In some embodiments, the recombinant anti-IL-23p19 antibody consists of a heavy chain of SEQ ID NO: 9 and a light chain of SEQ ID NO: 10.

In some embodiments, the dose of the anti-IL-23p19 antibody per administration is of 50-500 mg, preferably 50-300 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, or 500 mg. In some embodiments, the dose of the anti-IL-23p19 antibody per administration is of 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, or 250 mg. In a preferred embodiment, the dose administered each time during the dosing regimen is the same or different.

In some embodiments, the above method for treating moderate to severe psoriasis of the present disclosure comprises administering to a subject in need thereof an anti-IL-23p19 antibody, wherein the anti-IL-23p19 antibody is administered at 50-500 mg at Week 0, 4, and 8, and once every 12 weeks thereafter. In some preferred embodiments, 100-200 mg of the anti-IL-23p19 antibody is administered at Week 0, 4, and 8, and once every 12 weeks thereafter. In some more preferred embodiments, 200 mg of the anti-IL-23p19 antibody is administered at Week 0, 4, and 8, and once every 12 weeks thereafter.

In some embodiments, the above method of the present disclosure comprises administering to a subject in need thereof 200 mg of the anti-IL-23p19 antibody at Week 0, 4, and 8, followed by administering 50-300 mg of the anti-IL-23p19 antibody at Week 20 and/or Week 32 and/or Week 44. In a further embodiment, 50-250 mg of the anti-IL-23p19 antibody is administered at Week 20 and/or Week 32 and/or Week 44; in a still further specific embodiment, 100-200 mg of the anti-IL-23p19 antibody is administered to the subject in need thereof at Week 20 and/or Week 32 and/or Week 44. For example, 100 mg, 120 mg, 150 mg, 160 mg, 170 mg, 180 mg, 190 mg, or 200 mg of the anti-IL-23p19 antibody is administered to a patient in need thereof. Specifically, 100 mg or 200 mg of the anti-IL-23p19 antibody is administered to the subject in need thereof at Week 20 and/or Week 32 and/or Week 44.

In some embodiments, the above method of the present disclosure comprises administering to a subject in need thereof 200 mg of the anti-IL-23p19 antibody at Week 0, 4, and 8, administering 50-300 mg of the anti-IL-23p19 antibody at Week 20, then suspending administration of the anti-IL-23p19 antibody or continuing administration of a dose of 50-300 mg at Week 32 and 44, preferably administering 50-250 mg of the anti-IL-23p19 antibody at Week 20, 32, and 44, more preferably administering 100-200 mg of the anti-IL-23p19 antibody at Week 20, 32, and 44, and more preferably administering 100 mg or 200 mg of the anti-IL-23p19 antibody at Week 20, 32, and 44.

In some embodiments, the method described above of the present disclosure comprises administering to a subject in need thereof an anti-IL-23p19 antibody according to the following regimen:

1) administering 200 mg of the anti-IL-23p19 antibody at Week 0, 4, and 8, and administering 200 mg of the anti-IL-23p19 antibody at Week 20; or

2) administering 200 mg of the anti-IL-23p19 antibody at Week 0, 4, and 8, and administering 100 mg of the anti-IL-23p19 antibody at Week 20.

In some embodiments, the above method of the present disclosure further comprises detecting whether the subject responds to the treatment at Week 32. In some preferred embodiments, a subject having a response is defined as a PASI score improved by at least 90% relative to baseline, i.e., PASI90.

In some embodiments, the method described above of the present disclosure comprises administering to a subject who responds at Week 32 the anti-IL-23p19 antibody according to the following regimen:

1) administering 200 mg of the anti-IL-23p19 antibody at Week 0, 4, and 8, administering 100 mg of the anti-IL-23p19 antibody at Week 20, and administering 100 mg of the anti-IL-23p19 antibody at Week 32 and 44; or
2) administering 200 mg of the anti-IL-23p19 antibody at Week 0, 4, and 8, administering 100 mg of the anti-IL-23p19 antibody at Week 20, and then suspending administration of the anti-IL-23p19 antibody; or
3) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
4) 200 mg of the anti-IL-23p19 antibody was administered at Week 0, 4, and 8, 200 mg of the anti-IL-23p19 antibody was administered at Week 20, and then suspending administration of the anti-IL-23p19 antibody.

In some embodiments, the method described above of the present disclosure further comprises administering to a subject who does not respond at Week 32 an anti-IL-23p19 antibody according to the following regimen:

1) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
2) 200 mg of the anti-IL-23p19 antibody was administered at Week 0, 4, and 8, 100 mg of the anti-IL-23p19 antibody was administered at Week 20, and 200 mg of the anti-IL-23p19 antibody was administered at Week 32 and 44.

In some embodiments, the method comprises administering 200 mg of the anti-IL-23p19 antibody to a subject in need thereof at Weeks 0, 4, and 8, further administering 100 mg or 200 mg of the anti-IL-23p19 antibody at Week 20, and assessing whether the subject responds at Week 32, and

for subjects who respond at Week 32, administering 100 mg or 200 mg of the anti-IL-23p19 antibody at Week 32 and Week 44, respectively;

for subjects who do not respond at Week 32, administered 200 mg of the anti-IL-23p19 antibody at Week 32 and 44, respectively.

In the present disclosure, "response" is defined as at least a 90% improvement in PASI score from baseline, i.e., PASI90. PASI90 is defined as a $\geq$ 90% improvement in PASI score from baseline. The Psoriasis Area and Severity Index (PASI) is a systematic tool for assessing and grading the severity of psoriatic lesions and response to treatment.

In some embodiments, the method comprises:

1) 200 mg of the anti-IL-23p19 antibody is administered at Week 0, 4, and 8, 200 mg of the anti-IL-23p19 antibody is administered at Week 20, and when the subject is detected to be responsive at Week 32, 200 mg of the anti-IL-23p19 antibody is administered at Week 32 and 44; or
2) 200 mg of the anti-IL-23p19 antibody is administered at Week 0, 4, and 8, 200 mg of the anti-IL-23p19 antibody is administered at Week 20, and the administration of the anti-IL-23p19 antibody is no longer suspended when the subject is detected to be responsive at Week 32; or
3) 200 mg of the anti-IL-23p19 antibody is administered at Week 0, 4, and 8, 200 mg of the anti-IL-23p19 antibody is administered at Week 20, and 200 mg of the anti-IL-23p19 antibody is administered at Week 32 and 44 when the subject is detected to be unresponsive at Week 32; or
4) 200 mg of the anti-IL-23p19 antibody is administered at Week 0, 4, and 8, 100 mg of the anti-IL-23p19 antibody is administered at Week 20, and when the subject is detected to be responsive at Week 32, 100 mg of the anti-IL-23p19 antibody is administered at Week 32 and 44; or
5) 200 mg of the anti-IL-23p19 antibody is administered at Week 0, 4, and 8, 100 mg of the anti-IL-23p19 antibody is administered at Week 20, and discontinuing administration of the anti-IL-23p19 antibody when the subject is tested to be responsive at Week 32; or
6) 200 mg of the anti-IL-23p19 antibody was administered at Week 0, 4, and 8, 100 mg of the anti-IL-23p19 antibody was administered at Week 20, and 200 mg of the anti-IL-23p19 antibody was administered at Week 32 and 44 when the subject was detected to be non-responsive at Week 32.

In some embodiments, the method described above of the present disclosure further comprises detecting whether the subject has relapsed at Week 36. In some preferred embodiments, a subject is defined as having relapsed if their PASI response decreases by more than 50% between Week 36 and Week 44 relative to the response observed at Week 32.

In some embodiments, for subjects who responded at Week 32, the above method of the present disclosure further comprises detecting whether relapse occurs starting at Week 36. In some preferred embodiments, a subject is defined as having relapsed if the PASI response is lost by more than 50% relative to the Week 32 response between Week 36 and Week 44.

In some embodiments, for subjects who responded at Week 32 and continued to receive anti-IL-23p19 antibody, the above method of the present disclosure further comprises detecting whether relapse occurs starting at Week 36. In some preferred embodiments, a subject is defined as having relapsed if the PASI response is lost by more than 50% relative to the Week 32 response between Week 36 and Week 44.

In some embodiments, for subjects who responded at Week 32 but suspended administration of anti-IL-23p19 antibody after Week 20, the above method of the present disclosure further comprises detecting whether relapse occurs starting at Week 36. In some preferred embodiments, a subject is defined as having relapsed if the PASI response is lost by more than 50% relative to the Week 32 response between Week 36 and Week 44.

In some embodiments, for subjects who have relapsed, the above method of the present disclosure further comprises administering 200 mg of anti-IL-23p19 antibody once every four weeks starting from the time of confirmed relapse until the last dose at Week 44. In other embodiments, for subjects who responded at Week 32, if the PASI response is lost by more than 50% relative to the Week 32 response between Week 36 and Week 44 (relapse), 200 mg of anti-IL-23p19 antibody is administered to the subjects at the same dosing interval as that starting at Week 0 (200 mg of anti-IL-23p19 antibody is administered at Weeks 0, 4 and 8, followed by dosing once every 12 weeks thereafter) until the last dose at Week 44.

In some embodiments, the method comprises:

1) 200 mg of the anti-IL-23p19 antibody is administered at Week 0, 4, and 8, 200 mg of the anti-IL-23p19 antibody is administered at Week 20, and when the subject is detected to have a response at Week 32, 200 mg of the anti-IL-23p19 antibody is administered at Week 32 and 44; or

2) 200 mg of anti-IL-23p19 antibody is administered at Weeks 0, 4 and 8, 200 mg of anti-IL-23p19 antibody is administered at Week 20, and when the subject is detected to have a response at Week 32, administration of anti-IL-23p19 antibody is suspended; or

3) 200 mg of the anti-IL-23p19 antibody is administered at Week 0, 4, and 8, 200 mg of the anti-IL-23p19 antibody is administered at Week 20, and when the subject is detected to not have a respone at Week 32, 200 mg of the anti-IL-23p19 antibody is administered at Week 32 and 44; or

4) 200 mg of the anti-IL-23p19 antibody is administered at Week 0, 4, and 8, 100 mg of the anti-IL-23p19 antibody is administered at Week 20, and when the subject is detected to have a response at Week 32, 100 mg of the anti-IL-23p19 antibody is administered at Week 32 and 44; or

5) 200 mg of the anti-IL-23p19 antibody is administered at Week 0, 4, and 8, 100 mg of the anti-IL-23p19 antibody is administered at Week 20, and and when the subject is detected to have a response at Week 32, administration of anti-IL-23p19 antibody is suspended; or

6) 200 mg of the anti-IL-23p19 antibody is administered at Week 0, 4, and 8, 100 mg of the anti-IL-23p19 antibody is administered at Week 20, and when the subject is detected to not have a respone at Week 32, 200 mg of the anti-IL-23p19 antibody is administered at Week 32 and 44.

In a further embodiment, the above method further comprises:

for subjects receiving the administration regimen as defined in 1), detection of whether said subjects relapse starting at Week 36 is performed. A subject is defined as having relapsed if the PASI response is lost by more than 50% relative to the Week 32 response between Week 36 and Week 44, and for subjects who have relapsed, 200 mg of anti-IL-23p19 antibody is administered once every four weeks starting from the time of confirmed relapse until the last dose at Week 44;

for subjects receiving the administration regimen as defined in 2), detection of whether said subjects relapse starting at Week 36 is performed. A subject is defined as having relapsed if the PASI response is lost by more than 50% relative to the Week 32 response between Week 36 and Week 44, and for subjects who have relapsed, 200 mg of anti-IL-23p19 antibody is administered once every four weeks starting from the time of confirmed relapse until the last dose at Week 44;

for subjects receiving the administration regimen as defined in 4), detection of whether said subjects relapse starting at Week 36 is performed. A subject is defined as having relapsed if the PASI response is lost by more than 50% relative to the Week 32 response between Week 36 and Week 44, and for subjects who have relapsed, 200 mg of anti-IL-23p19 antibody is administered once every four weeks starting from the time of confirmed relapse until the last dose at Week 44; or

for subjects receiving the administration regimen as defined in 5), detection of whether said subjects relapse starting at Week 36 is performed. A subject is defined as having relapsed if the PASI response is lost by more than 50% relative to the Week 32 response between Week 36 and Week 44, and for subjects who have relapsed, 200 mg of anti-IL-23p19 antibody is administered once every four weeks starting from the time of confirmed relapse until the last dose at Week 44.

In some embodiments, the anti-IL-23p19 antibody of the present disclosure is formulated for administration in the form of a liquid pharmaceutical composition. Useful carriers and solvents include water, Ringer's solution, phosphate-buffered saline, isotonic sodium chloride solution, and the like. In addition, sterile, fixed oils may also be employed as a solvent or suspending medium where appropriate. Any mixed non-volatile mineral oil or non-mineral oil may be utilized for this purpose, including synthetic mono- or diglycerides. In addition, fatty acids such as oleic acid may also be used in the preparation of injectables.

In some embodiments, the pharmaceutical composition comprising the anti-IL-23p19 antibody of the present disclosure is a solution for injection or a dry powder formulation. For example, the composition is a lyophilized powder, which can be reconstituted into an injection solution in a pharmaceutically acceptable liquid carrier. The pharmaceutically acceptable liquid carrier may be, for example, sterile water, Ringer's solution, phosphate-buffered saline, isotonic sodium chloride solution, and the like.

In some embodiments, the pharmaceutical composition comprising the anti-IL-23p19 antibody of the present disclosure is a solution for injection, comprising or consisting of 100.0 mg/mL anti-IL23p19 antibody, 0.76 mg/mL histidine, 1.08 mg/mL histidine hydrochloride, 50.00 mg/mL sorbitol, and 0.5 mg/mL polysorbate 80, pH 6.0.

In some embodiments, the anti-IL-23p19 antibody or the pharmaceutical composition of the present disclosure is administered locally.

In some embodiments, the anti-IL-23p19 antibody or the pharmaceutical composition of the present disclosure is administered by subcutaneous injection.

In some embodiments, the anti-IL-23p19 antibody is recombinantly expressed in HEK 293 cells or CHO cells.

The formulation of the anti-IL-23p19 antibody of the present disclosure comprises 100.0 mg/mL anti-IL-23p19 antibody, 0.76 mg/mL histidine, 1.08 mg/mL histidine hydrochloride, 50.00 mg/mL sorbitol, 0.5 mg/mL polysorbate 80, at pH 6.0.

Preferably, the formulation of the IL-23p19 antibody can be prepared according to the method disclosed in PCT Application No. PCT/CN2021/093219 (International Application Date: May 12, 2021).

In some embodiments, the moderate to severe psoriasis is moderate to severe plaque psoriasis.

In some embodiments, the subject has moderate to severe plaque psoriasis for $\geq 6$ months, with or without psoriatic arthritis.

In some embodiments, the body surface area (BSA) affected by moderate to severe plaque psoriasis in the subject is $\geq 10\%$, the psoriasis area and severity index (PASI) is $\geq 12$ points, and the static physician's global assessment score (sPGA) is $\geq 3$ points.

In some embodiments of the above method, the subject does not experience a serious adverse event following administration.

In some embodiments of the above method, the incidence of adverse events in the subject is comparable to that in subjects receiving a placebo following administration.

In some embodiments of the above method, the subject is a human.

**Uses**

In a second aspect, the present disclosure relates to use of a recombinant anti-IL-23p19 antibody in the manufacture of a medicament for treating moderate to severe psoriasis in a subject.

In a third aspect, the present disclosure relates to a recombinant anti-IL-23p19 antibody for use in treating moderate to severe psoriasis.

For the second and third aspects, further embodiments thereof are as follows:

In some embodiments, the anti-IL23p19 antibody of the present disclosure comprises a heavy chain variable region and a light chain variable region, which further comprises the following 6 CDRs:

- heavy chain VH CDR1 as shown in GYTFTSYLMH (SEQ ID NO: 1);
- heavy chain VH CDR2 as shown in YINPYNEGTN (SEQ ID NO: 2);
- heavy chain VH CDR3 as shown in NWDLPY (SEQ ID NO: 3);
- light chain VL CDR1 as shown in RASQSISDYLH (SEQ ID NO: 4);
- light chain VL CDR2 as shown in YASQSMS (SEQ ID NO: 5); and
- light chain VL CDR3 as shown in QQGHSFPFT (SEQ ID NO: 6).

In some embodiments, the recombinant anti-IL-23p19 antibody comprises a heavy chain variable region VH and a light chain variable region VL, wherein the heavy chain variable region comprises the sequence of SEQ ID NO: 7 or a sequence having at least 90%, 95%, 98% or 99% identity thereto, and the light chain variable region comprises the sequence of SEQ ID NO: 8 or a sequence having at least 90%, 95%, 98% or 99% identity thereto.

In some embodiments, the recombinant anti-IL-23p19 antibody is an IgG1 antibody, preferably comprising a heavy chain sequence of SEQ ID NO: 9 or a sequence having at least 90%, 95%, 98%, or 99% identity thereto, and a light chain sequence of SEQ ID NO: 10 or a sequence having at least 90%, 95%, 98%, or 99% identity thereto.

In some embodiments, the recombinant anti-IL-23p19 antibody comprises a heavy chain set forth in SEQ ID NO: 9 and a light chain set forth in SEQ ID NO: 10. In some embodiments, the recombinant anti-IL-23p19 antibody consists of a heavy chain sequence of SEQ ID NO: 9 and a light chain sequence of SEQ ID NO: 10.

In some embodiments, the dose of anti-IL-23p19 antibody per administration is of 50-500 mg, preferably 50-300 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, or 500 mg. In some embodiments, the dose of anti-IL-23p19 antibody per administration is of 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, or 250 mg. In a preferred embodiment, the dose per administration is the same or different during the dosing regimen.

In some embodiments, the above uses of the present disclosure comprises administering 50-500 mg of the anti-IL-23p19 antibody at Week 0, 4, and 8, and administering once every 12 weeks thereafter. In some preferred embodiments, 100-200 mg of the anti-IL-23p19 antibody is administered at Week 0, 4, and 8, and once every 12 weeks thereafter. In some more preferred embodiments, 200 mg of the anti-IL-23p19 antibody is administered at Week 0, 4, and 8, and once every 12 weeks thereafter.

In some embodiments, the above use of the present disclosure comprises administering to a subject in need thereof 200 mg of the anti-IL-23p19 antibody at Week 0, 4, and 8, and administering 50-300 mg of the anti-IL-23p19 antibody at Week 20 and/or 32 and/or 44. In a further embodiment, 50-250 mg of the anti-IL-23p19 antibody is administered at Week 20 and/or Week 32 and/or Week 44; in a still further specific embodiment, 100-200 m g of the anti-IL-23p19 antibody is administered to the subject in need thereof at Week 20 and/or Week 32 and/or Week 44. For example, 100 mg, 120 mg, 150 mg, 160 mg, 170 mg, 180 mg, 190 mg, or 200 mg of the anti-IL-23p19 antibody is administered to a patient in need thereof. Specifically, 100 mg or 200 mg of the anti-IL-23p19 antibody is administered to the subject in need thereof at Week 20 and/or Week 32 and/or Week 44.

In some embodiments, the above method of the present disclosure comprises administering to a subject in need thereof 200 mg of anti-IL-23p19 antibody at Weeks 0, 4 and 8, administering 50 mg-300 mg of anti-IL-23p19 antibody at Week 20, and then suspending administration of anti-IL-23p19 antibody or continuing to administer 50 mg-300 mg of anti-IL-23p19 antibody at Weeks 32 and 44, preferably administering 50 mg-250 mg of anti-IL-23p19 antibody at Weeks 20, 32 and 44, more preferably administering 100 mg-200 mg of anti-IL-23p19 antibody at Weeks 20, 32 and 44, and more preferably administering 100 mg or 200 mg of anti-IL-23p19 antibody at Weeks 20, 32 and 44.

In some embodiments, the above uses of the present disclosure comprise administering an anti-IL-23p19 antibody to a subject in need thereof according to the following regimen.:

1) administering 200 mg of the anti-IL-23p19 antibody at Week 0, 4, and 8, and administering 200 mg of the anti-IL-23p19 antibody at Week 20; or
2) administering 200 mg of the anti-IL-23p19 antibody Week 0, 4, and 8, and administering 100 mg of the anti-IL-23p19 antibody at Week 20.

In some embodiments, the above uses of the present disclosure further comprise detecting whether the subject responds to the treatment at Week 32. In some preferred embodiments, a subject having a response is defined as a PASI score improved by at least 90% relative to baseline, i.e., PASI90.

In some embodiments, the above uses of the present disclosure comprise administering to a subject who responds at Week 32 the anti-IL-23p19 antibody according to the following regimen:

1) administering 200 mg of the anti-IL-23p19 antibody at Week 0, 4, and 8, administering 100 mg of the anti-IL-23p19 antibody at Week 20, and administering 100 mg of the anti-IL-23p19 antibody at Week 32 and 44; or
2) administering 200 mg of the anti-IL-23p19 antibody at Week 0, 4, and 8, administering 100 mg of the anti-IL-23p19 antibody at Week 20, and then suspending administration of the anti-IL-23p19 antibody; or
3) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
4) administering 200 mg of the anti-IL-23p19 antibody at Week 0, 4, and 8, administering 200 mg of the anti-IL-23p19 antibody at Week 20, and then suspending administration of the anti-IL-23p19 antibody.

In some embodiments, the above use of the present disclosure further comprises detecting whether a subject relapses or not at Week 36. In some preferred embodiments, a subject is defined as having relapsed if the PASI response is lost by more than 50% relative to the Week 32 response between Week 36 and Week 44.

In some embodiments, for a subject who has responded at Week 32, the above use of the present disclosure further comprises detecting whether the subject has relapsed since Week 36. In some preferred embodiments, a subject is defined as having relapsed if the PASI response is lost by more than 50% relative to the Week 32 response between Week 36 and Week 44.

In some embodiments, for a subject who has responded at Week 32 and continues to receive administration of an anti-IL-23p19 antibody, the use described above of the present disclosure further comprises detecting whether the subject has relapsed starting at Week 36. In some preferred embodiments, a subject is defined as having relapsed if the PASI response is lost by more than 50% relative to the Week 32 response between Week 36 and Week 44.

In some embodiments, for a subject who responds at Week 32 but suspends administration of the anti-IL-23p19 antibody after Week 20, the above use of the present disclosure further comprises detecting whether the subject has relapsed starting at Week 36. In some preferred embodiments, a subject is defined as having relapsed if the PASI response is lost by more than 50% relative to the Week 32 response between Week 36 and Week 44.

In some embodiments, the above uses of the present disclosure further comprise administering 200 mg of anti-IL-23p19 antibody to subjects who have relapsed once every four weeks starting from the time of confirmed relapse until the last dose at Week 44.

In some embodiments, the use described above of the present disclosure further comprises administering to a subject who does not respond at Week 32 an anti-IL-23p19 antibody according to the following regimen:

1) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
2) administering 200 mg of the anti-IL-23p19 antibody at Week 0, 4, and 8, administering 100 mg of the anti-IL-23p19 antibody at Week 20, and administering 200 mg of the anti-IL-23p19 antibody at Week 32 and 44.

In some embodiments, the use comprises administering to a subject in need thereof 200 mg of the anti-IL-23p19 antibody at Week 0, 4, and 8, 100 mg or 200 mg of the anti-IL-23p19 antibody at Week 20, and 100 mg or 200 mg of the anti-IL-23p19 antibody at Week 32 and 44 to a subject who responds at Week 32.

wherein the use comprises administering to a subject in need thereof 200 mg of the anti-IL-23p19 antibody at Week 0, 4, and 8, 100 mg or 200 mg of the anti-IL-23p19 antibody at Week 20, and 200 mg of the anti-IL-23p19 antibody at Week 32 and 44 to a subject who is a non-responder at Week 32, respectively.

**Single pharmaceutical dosage unit**

In a fourth aspect, the present disclosure relates to a single pharmaceutical dosage unit comprising the anti-IL23p19 antibody of the present disclosure.

In some embodiments, the single pharmaceutical dosage unit comprises the anti-IL-23p19 antibody at a fixed dose of 50-500 mg, more preferably 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, or 500 mg.

In some embodiments, the single pharmaceutical dosage unit is a single pharmaceutical dosage unit packaged in a prefilled automated injection pen.

**Pharmaceutical kit**

The present disclosure further provides a pharmaceutical kit, comprising an effective amount of the anti-IL-23p19 antibody described above; preferably, comprising a fixed dose of 50 mg-500 mg; more preferably, comprising 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, or 500 mg of the anti-IL-23p19 antibody;

Specifically, a package insert is also included with instructions for using the anti-IL-23p19 antibody to treat moderate to severe psoriasis in a subject.

In some embodiments, the pharmaceutical kit comprises the anti-IL-23p19 antibody in a single pharmaceutical dosage unit form. In other embodiments, the single pharmaceutical dosage unit is packaged in a prefilled automatic injection pen.

**Applications**

The present disclosure further provides use of the anti-IL-23p19 antibody, the single pharmaceutical dosage unit, and the pharmaceutical kit of the present disclosure in the manufacture of a medicament for treating moderate to severe psoriasis.

The method for treating moderate to severe psoriasis with the anti-IL-23p19 antibody of the present disclosure has the characteristics of long half-life and long interval administration, and is administered once every 12 weeks after stable treatment, so that it has the potential to maintain the efficacy for a longer time after drug withdrawal, and the number of patients who maintain the efficacy within the same time after drug withdrawal is larger.

**EXAMPLES**

**Example 1. Preparation and purification of IL-23p19 antibody**

The antibody 17D1-YTE specifically binding to IL-23p19 was obtained as described in PCT Application No. PCT/CN2019/121261. The antibody has a heavy chain sequence of SEQ ID NO:9 and a light chain sequence of SEQ ID NO:10. PCT Application No. PCT/CN2019/121261 is hereby incorporated herein by reference in its entirety.

Briefly, the antibody was recombinantly expressed in CHO cells and purified by affinity chromatography to give the IL-23p19 antibody sample used in the pH screening assay of the present disclosure, and the IL-23p19 antibody sample used in the formula screening assay of the present disclosure was purified by cation exchange chromatography.

**Example 2. Clinical Studies**

The results of preclinical pharmacokinetics (PK), pharmacodynamics (PD), and toxicology studies showed that the anti-IL-23P19 antibody had a clear target, a clear mechanism of action, and significant anti-inflammatory effects, and was expected to reduce or clear the skin lesions of subjects with plaque psoriasis, improve the severity of the skin lesions of the subjects, and reduce the cumulative body surface area of the skin lesions. The anti-IL-23P19 antibody exhibited linear PK characteristics in cynomolgus monkeys, with a long half-life, high bioavailability, high in vivo safety after short-term or long-term repeated administration in cynomolgus monkeys, and no risk of hemolysis or cytokine release in vitro.

The randomized, double-blind, placebo-controlled, phase I clinical study on the safety and tolerability of single-dose escalation in healthy subjects has been completed for the anti-IL-23p19 antibody of the present application. The results show that the anti-IL-23P19 antibodies were safe and well tolerated in healthy humans.

The anti-IL-23p19 antibody of the present application has completed a dose-ranging phase II multicenter, randomized, double-blind, placebo-controlled clinical study in moderate to severe plaque psoriasis and has reached the primary efficacy endpoint. The results of the phase II study showed that the anti-IL-23p19 monoclonal antibody could significantly improve the skin lesions of subjects with moderate to severe plaque psoriasis and had good overall safety and tolerability. Moreover, the 52-week data show the advantages of the anti-IL-23P19 antibody in terms of long-term efficacy, and meanwhile, the anti-IL-23P19 antibody significantly improved the quality of life of the subjects compared with the placebo group.

The above results support further clinical studies of the anti-IL-23P19 antibody.

This study is a multicenter, randomized, double-blind, placebo-controlled, randomized withdrawal and retreatment phase III study of anti-IL-23P19 antibodies. The primary objectives are to evaluate the maintenance efficacy of anti-IL-23P19 antibody maintenance therapy relative to withdrawal and to understand the time after withdrawal when the efficacy of anti-IL-23P19 antibody can be maintained. This is also the first randomized withdrawal retreatment design phase III study of drugs with the same target in China, so as to provide more extensive confirmatory data support for the efficacy and safety of anti-IL-23P19 antibody in subjects with moderate to severe plaque psoriasis.

### 3.1 Study Objectives

- Primary Objective:

  - To evaluate the maintenance of efficacy of the anti-IL-23P19 antibodies after randomized withdrawal in the treatment of moderate to severe plaque psoriasis.

- Secondary objective:

- To evaluate the efficacy of the anti-IL-23P19 antibody in the retreatment of subjects with moderate to severe plaque psoriasis who relapse after randomized withdrawal;
- To evaluate the safety of the anti-IL-23P19 antibody in the treatment of moderate to severe plaque psoriasis;
- To evaluate the immunogenicity of the anti-IL-23P19 antibodies in patients with moderate to severe plaque psoriasis;
- To evaluate the impact of the anti-IL-23P19 antibodies on health-related quality of life in patients with moderate to severe plaque psoriasis.

- Other objectives:

  - To evaluate the efficacy of the anti-IL-23P19 antibodies in the treatment of scalp psoriasis in patients with baseline scalp psoriasis;
  - To evaluate the efficacy of the anti-IL-23P19 antibody in the treatment of nail psoriasis in patients with baseline nail psoriasis;
  - To evaluate the efficacy of the anti-IL-23P19 antibody in the treatment of patients with baseline palmoplantar psoriasis for palmar-plantar psoriasis;
  - To evaluate the efficacy of the anti-IL-23P19 antibody in treating patients with baseline perineal psoriasis for perineal psoriasis;
  - To explore the efficacy of the anti-IL-23P19 antibodies on baseline PsA activity score in patients with psoriatic arthritis (PsA).

### 3.2 Study Endpoints

- Primary endpoint:

  - Proportion of subjects who achieve ≥90% improvement in Psoriasis Area and Severity Index (PASI 90) at Week 56.

- Secondary endpoints:
  Secondary Efficacy Endpoints

  - Proportion of subjects who achieve a ≥75% improvement in PASI (PASI 75) at Week 56;
  - Proportion of subjects who achieve a 100% improvement in PASI (PASI 100) at Week 56;
  - The proportion of subject who achieved sPGA clean (0) or near clean (1) at Week 56.
  - Proportion of subject achieving sPGA clean (0) at Week 56;
  - Proportion of subjects with DLQI score of 0/1 at Week 56;
  - Time to loss of PASI90 in subjects;
  - Change in PASI and sPGA in retreated subjects with relapse

Safety endpoints

All AEs (Adverse Event), SAEs (Serious Adverse Event), etc. Changes in vital signs, physical examination, laboratory tests, Electrocardiography (ECG), etc. before and after administration.

Immunogenicity evaluation

Generation of anti-drug antibodies (ADAs) and neutralizing antibodies (NAbs).

- Other endpoints:

  - Change in DLQI from baseline at different time points;
  - Proportion of subjects achieving PASI 90, PASI 75, PASI 100, sPGA 0 or sPGA 0/1 at different time points, and proportion of subjects achieving DLQI 0/1 at different time points;
  - Change from baseline in PSSI over time (limited to subjects with concomitant scalp psoriasis at baseline);
  - Change from baseline in NAPSI over time (limited to subjects with concomitant nail psoriasis at baseline);
  - Change in PPASI from baseline over time (limited to subjects with concomitant palmoplantar psoriasis at baseline);

- Change from baseline in sPGA-G over time (limited to subjects with concomitant perineal psoriasis at baseline);
- Change from baseline in Psoriatic Arthritis Disease Activity Score over time (limited to subjects with concomitant psoriatic arthritis at baseline).

### 3.3 Study Design

This study adopted a multicenter, randomized, double-blind, placebo-controlled, randomized withdrawal and retreatment design, and mainly evaluated the maintenance of efficacy after random drug withdrawal in the treatment of moderate to severe plaque psoriasis with subcutaneous injection of anti IL-23P19 antibody.. The target population will be men or women 18 to 75 years of age with a diagnosis of plaque psoriasis for at least 6 months prior to the first dose of study drug, with or without psoriatic arthritis. Subjects diagnosed with plaque psoriasis must have sPGA $\geq$ 3, PASI $\geq$ 12, and involved body surface area (BSA) $\geq$ 10%.

Approximately 550 patients with moderate to severe plaque psoriasis were planned to be enrolled.

After 4 weeks of screening, eligible subjects received 200 mg of anti-IL-23p19 antibody by subcutaneous injection at Week 0, 4, and 8. At Week 20, the subjects were randomized into 2 groups in a 1:1 ratio to receive 200 mg of anti-IL-23p19 antibody or 100 mg of anti-IL-23p19 antibody, with previous treatment with biologics as a stratification factor.

All subjects in each group were assessed for whether response to treatment at Week 32 (response was defined as an ASI90 of at least 90% improvement in PASI score from baseline). Subjects with responses in each group were then re-randomized, i.e., subjects with responses in the group that had previously received 200 mg of the anti-IL-23p19 antibody were re-randomized in a 1:1 ratio into 2 groups, one group continued to receive treatment with 200 mg of the anti-IL-23p19 antibody (i.e., the 200 mg maintenance treatment group) and one group received placebo (i.e., the 200 mg withdrawal group); subjects with responses in the group that had previously received 100 mg of the anti-IL-23p19 antibody were re-randomized in a 1:1 ratio into 2 groups, one group continued to receive treatment with 100 mg of the anti-IL-23p19 antibody (i.e., the 100 mg maintenance treatment group) and one group received placebo (i.e., the 100 mg withdrawal group). In all groups, corresponding drugs were administered once every 12 weeks from Week 32, and the last dose was administered at Week 44. For subjects who did not respond at Week 32 in the groups that received 200 mg of anti-IL-23p19 antibody or 100 mg of anti-IL-23p19 antibody at Week 20, 200 mg of anti-IL-23p19 antibody was administered at Week 32 and 44, respectively.

Subjects assessed as responsive to treatment at Week 32 and re-randomized will be referred to as relapsers if their PASI response decreases by more than 50% from Week 32 PASI response between Week 36 and Week 44. For relapsers, 200 mg of anti-IL-23p19 antibody was administered once every four weeks from the time of relapse determination until the last dose at Week 44; if the PASI response at Week 48 was reduced by more than 50% relative to the PASI response at Week 32, the subject was withdrawn from the study.

The last dose was administered at Week 44 and ended at the last follow-up visit at Week 56. Completion of the Week 56 visit of the study will be considered completion of the study.

### 3.4 Inclusion Criteria

Eligible subjects must meet all of the following inclusion criteria:

(1) Male or female aged 18 to 75 years;
(2) Diagnosed with plaque psoriasis for more than 6 months, with or without psoriatic arthritis;
(3) Body surface area (BSA) affected by plaque psoriasis $\geq$ 10%, psoriasis area and severity index (PASI) $\geq$ 12, and static physician's global assessment score (sPGA) $\geq$ 3 at screening and baseline;
(4) The subject is suitable for phototherapy and/or systemic treatment of psoriasis;
(5) Fully understand the purpose of the test, basically understand the pharmacological effects of the test drug and the possible adverse reactions; voluntarily sign the informed consent according to the spirit of the Declaration of Helsinki.

### 3.5 Exclusion Criteria

Eligible subjects did not meet any of the following exclusion criteria:

(1) Diagnosed with other types of psoriasis other than non-plaque psoriasis (guttate psoriasis, pustular psoriasis, or erythrodermic psoriasis);
(2) Diagnosis of drug-induced psoriasis (e.g., psoriasis caused by $\beta$ blockers, calcium channel inhibitors, etc.);
(3) Patients who have previously used anti-IL23P19 antibodies, or IL-23 target therapy;
(4) Received local treatment drugs that may affect the evaluation of psoriasis (including but not limited to glucocorticoids, vitamin D3 derivatives, retinoids, calcineurin inhibitors, keratolytic agents, and compound preparations) within 2 weeks before the first use of the study drug;

(5) Received systemic drug therapy that may affect the evaluation of psoriasis within 4 weeks before the first use of the study drug (including but not limited to methotrexate, cyclosporine, retinoids, azathioprine, leflunomide, mycophenolate mofetil, sulfasalazine, glucocorticoids, JAK inhibitors such as tofacitinib, baricitinib, or traditional Chinese medicine or proprietary Chinese medicine for the treatment of psoriasis);

(6) Received tumor necrosis factor-$\alpha$ (TNF-$\alpha$) antagonists (including but not limited to etanercept, infliximab, and adalimumab) within 3 months (or within 5 half-lives of the drug) before the first use of the study drug;

(7) Received IL-17 target treatment (including but not limited to secukinumab, ixekizumab, etc.) within 6 months (or within 5 half-lives of the drug) before the first use of the study drug;

(8) Use of natalizumab, or B-cell or T-cell modulators (e.g., rituximab, Abatacept, or Visilizumab) within 12 months prior to the first dose;

(9) Received phototherapy for psoriasis within 1 month prior to the first dose, and/or is unwilling to avoid continuous direct sunlight exposure and other ultraviolet light sources during the study;

(10) There is evidence that the subject has severe, progressive, uncontrolled (including but not limited to) cardiovascular disease, neuromuscular disease, blood disease, respiratory disease, liver or digestive disease, urinary disease, neurological or psychiatric disease;

(11) Opportunistic infections [such as herpes zoster (severe or recurrent), active cytomegalovirus, pneumocystis carinii, histoplasma, aspergillus, mycobacteria, etc.] within 6 months before screening;

(12) Known history of recurrent or chronic infection, history of chronic or recurrent infection, including but not limited to: chronic renal infection, chronic chest infection (such as bronchiectasis), recurrent urinary tract infection, open, draining or skin infectious wounds;

(13) have a history of serious infection (e.g., sepsis, pneumonia, pyelonephritis), or have been hospitalized or received intravenous antibiotics for infection within 2 months prior to screening;

(14) A history of malignant tumor or malignancy (except for squamous cell carcinoma of the skin, basal cell carcinoma, or localized cervical carcinoma in situ that has been successfully resected and has no evidence of recurrence or metastasis within 5 years);

(15) Suffering from or have suffered from lymphoproliferative disease, or have symptoms or signs suggestive of lymphoproliferative disease within 5 years before screening, such as lymph nodes and/or splenomegaly;

(16) Patients with a known history of active tuberculosis or suspected tuberculosis based on clinical manifestations (including but not limited to pulmonary tuberculosis, lymphoid tuberculosis, tuberculous pleurisy, etc.); the tuberculosis status of the subjects was screened by interferon gamma release assay (IGRA) and anteroposterior and lateral chest X-ray. For subjects without symptoms of active tuberculosis and imaging evidence of tuberculosis:

- Negative IGRA test result, meeting eligibility;
- Patients with indeterminate IGRA test results who can be retested and still have indeterminate retest results do not meet the eligibility criteria;
- Positive IGRA test results may be re-screened and evaluated after no less than 1 month of prophylactic anti-tuberculosis treatment. Patients without symptoms of tuberculosis and with good tolerance to tuberculosis drugs, who are willing to receive complete prophylactic anti-tuberculosis treatment during the study period may be enrolled as assessed by the investigator.

(17) BCG vaccination within 12 months before the first use of the study drug, or plan to receive BCG vaccination during the study period or within 12 months after the last study treatment;

(18) Receive live or bacterial vaccines within 3 months prior to the first dose of the study drug, or plan to receive live or bacterial vaccines during the study period or within 3 months after the last study treatment;

(19) Received treatment with an experimental biological agent within 6 months prior to the first dose of the study drug, or received any experimental treatment within 30 days, or received the study drug within 5 half-lives, or is participating in a clinical study;

(20) The results of blood routine and blood biochemistry tests during the screening period and baseline period meet the following conditions:

- Any parameter of hemoglobin, red blood cells, white blood cells, neutrophils, and platelets < lower limit of normal value (LLN) and abnormal with clinical significance as judged by the investigator;
- Any parameter of alanine aminotransferase (ALT), aspartate aminotransferase (AST), total bilirubin (TBIL), or direct bilirubin (DBIL) > 2 times the upper limit of normal value (ULN);
-

## Creatinine (Cr) > ULN;

Subjects who meet the protocol requirements after re-examination may also be enrolled.

(21) The virus test results at the time of screening should meet any of the following criteria:

Human immunodeficiency virus (HIV) antibody positive;
Hepatitis C virus (HCV) antibody positive with no history of successful treatment, with successful treatment defined as negative HCV RNA at least 24 weeks after completion of antiviral treatment;
Hepatitis B virus (HBV) screening includes at least hepatitis B surface antigens (HBsAg), hepatitis B surface antibodies (HBsAb), and hepatitis B core antibodies (HBcAb), and if the test results of the above three indicators are: HBsAg positive; or if only HBcAb is positive when HBsAg is negative, HBV DNA should be tested and the test result is positive;

- Syphilis-specific antibody positive (except for those with syphilis non-specific antibody titer turning negative after regular syphilis treatment).

(22) At screening, have the following clinically significant 12-lead ECG abnormalities: QTcF > 450 ms, shortened or prolonged PR interval, second- or third-degree atrioventricular block, pre-excitation syndrome, long QT syndrome; or severe arrhythmia requiring treatment;
(23) Those who have had severe drug and food allergies in the past, and/or those who are allergic to the test drug or its components;
(24) History of alcohol and drug abuse within 12 months prior to screening;
(25) Female subjects who are pregnant or breastfeeding, or women of gestational age who have a positive pregnancy test at screening and before administration;
(26) Those who plan to have children during the study period and within 6 months after the study drug administration, or are unwilling to take appropriate contraceptive measures (such as condoms) during the study period as deemed by the doctor;
(27) The researcher believes that it is not suitable to participate in this clinical trial for various reasons.

### 3.6 Study Drug Dose and Method of Administration

At Week 0, 4, and 8, the subjects received subcutaneous injections of 200 mg of anti-IL23P19 antibody; at Week 20, the subjects were randomized in a 1:1 ratio to receive subcutaneous injections of 200 mg of anti-IL23P19 antibody or 100 mg of anti-IL23P19 antibody. At Week 32, responders (those who achieve PASI 90) in each group will undergo a second randomization (re-randomization).
Subjects who were in response and received 200 mg of anti-IL23P19 antibody at Week 20 were divided into a 200 mg maintenance treatment group and a 200 mg withdrawal group. Subjects in the 200 mg maintenance treatment group received 200 mg anti-IL23P19 antibody at Week 32 and 44, respectively, with the last dose at Week 44; subjects in the 200 mg withdrawal group received the corresponding placebo at Week 32 and 44, respectively, with the last dose at Week 44.
Subjects in response who received 100 mg anti-IL23P19 antibody at Week 20 were divided into a 100 mg maintenance treatment group and a 100 mg withdrawal group. Subjects in the 100 mg maintenance treatment group received 100 mg anti-IL23P19 antibody at Week 32 and 44, respectively, with the last dose at Week 44; subjects in the 100 mg withdrawal group received the corresponding placebo at Week 32 and 44, respectively, with the last dose at Week 44.
Subjects tested for non-response at Week 32 all received 200 mg anti-IL23P19 antibody at Week 32 and 44.
Subjects who were tested to be responders at Week 32 but relapsed at Week 36 to 44 received subcutaneous injection of 200 mg of anti-IL23P19 antibody once every 4 Week for a total of three doses at the time of relapse.
Specifically :

100 mg-maintenance treatment group: subcutaneous administration of 200 mg of anti-IL-23P19 antibody at Week 0, 4, and 8, subcutaneous administration of 100 mg of anti-IL-23P19 antibody at Week 20, and every 12 Week thereafter until Week 44 ( last dose );
100 mg-withdrawal/placebo group: 200 mg of anti-IL-23P19 antibody administered subcutaneously at Week 0, 4, and 8, 100 mg of anti-IL-23P19 antibody administered subcutaneously at Week 20, and placebo administered every 12 Week thereafter until Week 44 ( last dose );
200 mg-maintenance treatment group: subcutaneous administration of 200 mg of anti-IL-23P19 antibody at Week 0, 4, and 8, subcutaneous administration of 200 mg of anti-IL-23P19 antibody at Week 20, and every 12 Week thereafter until Week 44 ( last dose );
200 mg-withdrawal/placebo group: 200 mg of anti-IL-23P19 antibody administered subcutaneously at Week 0, 4, and 8, 200 mg of anti-IL-23P19 antibody administered subcutaneously at Week 20, and placebo administered every 12 Week thereafter until Week 44 ( last dose ).

For subjects who are detected to have a response at Week 32 and no longer receive an anti-IL-23P19 antibody after Week 20, those who lose more than 50% of the PASI response between Week 36 and Week 44 relative to the PASI response at Week 32 are referred to as relapsers. The relapser was given 200 mg of anti-IL-23p19 antibody at relapse once every 4 weeks for a total of three doses until the last dose at Week 44. Subjects will be withdrawn from the study if their PASI response decreases by more than 50% from Week 48 compared to Week 32.

For subjects who did not respond at Week 32, 200 mg of anti-IL23P19 antibody was administered at Week 32 and 44.

### 3.7 Treatment Compliance

The subjects received the study treatment at the study site, and their treatment compliance was monitored using drug dispensation records, subject medical records, and eCRFs.

### 3.8 Indicators related to efficacy evaluation

3.8.1. PASI

The Psoriasis Area and Severity Index (PASI) is a systematic tool for assessing and grading the severity of psoriatic skin lesions and response to treatment. PASI scores range from 0 to 72.

Every effort should be made to ensure that the physician who performed the PASI assessment on the subject at screening/baseline conducts the PASI assessment for the same subject during subsequent follow-up visits.

PASI90 refers to an improvement in PASI score of $\geq$ 90% from baseline; PASI 75 refers to an improvement in PASI score of $\geq$ 75% from baseline; PASI100 refers to an improvement in PASI score of 100% from baseline.

Response is defined as at least a 90% improvement in PASI score from baseline, i.e., PASI90.

Relapse was defined as a loss of more than 50% of PASI response relative to Week 32 response.

3.8.2. sPGA

The static Physician's Global Assessment (sPGA) records the physician's assessment of the subject's psoriasis status, including the following: induration, scaling, and erythema. Whenever possible, the physician performing the sPGA assessments at Screening/Baseline performed the sPGA assessments for the subject at subsequent visits.

3.8.3. DLQI

The Dermatology Life Quality Index (DLQI) is a dermatology-related quality-of-life instrument designed to assess the impact of a disease on a participant's quality of life (AY Finlay and GK Khan). A 10-question questionnaire assessing 6 different aspects of quality of life: symptoms and feelings, daily activities, leisure activities, work or school standards, personal relationships and treatment.

For visits involving the DLQI assessment, the DLQI assessment was performed before all other visit contents (examination, procedures, psoriasis assessment, collection of adverse events and concomitant medications, etc.) during the study.

3.8.4. PSSI

The Psoriasis Scalp Severity Index (PSSI) assessment is only used for subjects with concomitant scalp psoriasis at baseline. The PSSI records the range of scalp psoriasis, and also records the induration, scaling, and erythema of scalp skin lesions of scalp psoriasis. Similar to PASI, the numerical score ranges from 0 to 72. Every effort should be made to ensure that the physician who performed the PSSI assessment on the subject at screening/baseline conducts the PSSI assessment for the same subject during subsequent follow-up visits.

3.8.5. NAPSI

Nail Psoriasis Severity Index (NAPSI) assessments are only used for subject with concomitant nail psoriasis at baseline. NAPSI Evaluation of Nail matrix and Nail bed damage with a total score ranging from 0 to 80. Every effort should be made to ensure that the physician who performed the NAPSI assessment on the subject at screening/baseline conducts the NAPSI assessment for the same subject during subsequent follow-up visits.

3.8.6. PPASI

Palmar-Plantar Psoriasis Severity Index (PPASI) assessment will be used only for subjects with concomitant palmoplantar psoriasis at baseline. PASI evaluation of palmar and plantar psoriasis, with a total score ranging from 0-72. Every effort should be made to ensure that the physician who performed the PPASI assessment on the subject at screening/baseline conducts the PPASI assessment for the same subject during subsequent follow-up visits.

3.8.7. sPGA-G

sPGA-G assessments are only used for subjects with concomitant perineal psoriasis at baseline. The sPGA-G is a local evaluation of perineal psoriasis using the evaluation criteria of sPGA. Every effort should be made to ensure that the physician who performed the sPGA-G assessment on the subject at screening/baseline conducts the sPGA-G assessment for the same subject during subsequent follow-up visits.

3.8.8. PsA disease activity

Psoriatic Arthritis Disease Activity Assessment is only used for subject with concomitant psoriatic arthritis at baseline. The physician and the subject must complete this assessment independently. Every effort should be made to ensure that the physician who performed the PsA assessment on the subject at screening/baseline conducts the PsA assessment for the same subject during subsequent follow-up visits.

Visual Analogue Scales (VAS) was used to record assessments of psoriatic arthritis disease activity by "subjects" and "doctors". The subject's assessment on the VAS ranged from "very good (0 cm)" to "very bad (10 cm)" and the physician's assessment on the VAS ranged from "no arthritic activity (0 cm)" to "arthritis and its activity (10 cm)".

3.8.9 Skin lesion photography

In order to obtain more data for efficacy evaluation, when conducting efficacy assessment each time, take photos of the skin condition of the subjects (excluding the perineum).

**3.9 Safety Assessment**

I. Laboratory Tests

Routine laboratory safety assessments include the laboratory tests listed in the table below.

Contents of routine laboratory tests

| Hematology | Red blood cell (RBC) count, hemoglobin (HGB), white blood cell (WBC) count, platelet (PLT) count, neutrophil percentage, lymphocyte percentage, monocyte percentage. |
|---|---|
| Blood biochemistry | Aspartate Aminotransferase (AST), Alanine Aminotransferase (ALT), Total Bilirubin (TBIL), Direct Bilirubin (DBIL), Total Protein (TP), Albumin (ALB), Fasting Blood Glucose (FBG), Alkaline Phosphatase, Serum Potassium, Serum Sodium, Serum Calcium, Serum Phosphorus, Uric Acid, Triglyceride (TG), Lactate Dehydrogenase (LDH), Creatine Kinase (CK), Urea (UREA)/Blood Urea Nitrogen (BUN), And Creatinine (Cr). |
| Urinalysis | Urine Protein (UPRO), Urine Glucose (UGLU), Urinary Red Blood Cells (URBC), Urinary White Blood Cells (UWBC), Ketone (KET), Specific Gravity (SG), Uric Alkalinity (Ph), Urobilinogen (URO), and Urinary Nitrite (NIT). |
| Viral serology | Hepatitis B panel: HBsAg, HBsAb, HBcAb, HBeAb, and HBeAg; HCV antibody and HIV antibody. If necessary, HBV-DNA, HCV-RNA; syphilis-specific antibodies and non-specific antibodies may be tested. Eligibility requirements for hepatitis B test indicators: |

| HBsAg | HBsAb | HBcAb | ELIGIBILITY |
|---|---|---|---|
| + | + (or -) | + (or -) | Not compliant |
| - | + | + (or -) | Complies |
| - | - | + | Detect HBV-DNA; the result below the lower limit of quantitation shall comply with the requirements |
| - | - | - | Complies |

| Tuberculosis test | Interferon gamma release assays (IGRAs) include QuantiFERON-TB or T-SPOT. |
|---|---|
| Pregnancy test | Blood pregnancy test or urine pregnancy test. |

HBcAb, hepatitis B core antibody; HBeAb, hepatitis B e antibody; HBeAg, hepatitis B e antigen; HBsAb, hepatitis B surface antibody; HBsAg, hepatitis B surface antigen; HBV, hepatitis B virus; HCV, hepatitis C virus; HIV, human immunodeficiency virus.

Relevant laboratory tests will be performed under fasting conditions.

II. Clinical Examination

Clinical examinations include general physical examination, vital signs (including temperature, pulse, and blood pressure), 12-lead electrocardiogram (12-lead ECG), chest radiography, serious allergic reactions, injection site reactions, cardiovascular events (including major cardiovascular events (e.g., cardiovascular- and cerebrovascular-related death, non-fatal myocardial infarction, and non-fatal stroke) and other cardiovascular events known in the art), and psychiatric-related events.

3.10 Immunogenicity

About 5 mL of whole blood was collected, and the serum was separated, aliquoted, and cryopreserved for ADA and NAb analysis.

### 3.11 Statistical Analysis Methods

3.11.1 Hypothesis Testing

The primary endpoint of this trial is a dual-endpoint design: Compare the proportions of subjects with a $\geq 90\%$ improvement in PASI at Week 56 between the 100 mg maintenance treatment group and the 200 mg maintenance treatment group respectively and the re-randomized placebo group. Only when both endpoints are achieved simultaneously is it considered statistically significant. Therefore, no $\alpha$ adjustment is made.
Primary endpoint:

- Proportion of subjects in the 100 mg maintenance treatment group p11, proportion of subjects in the 100 mg withdrawal/placebo group p10:

$$H0: p11 = p10 \quad H1: p11 \neq p10$$

- Proportion of subjects in the 200 mg maintenance treatment group p21, proportion of subjects in the 200 mg withdrawal/placebo group p20:

$$H0: p21 = p20 \quad H1: p21 \neq p20$$

After primary dual endpoints achieve positive results, conduct sequential tests for secondary efficacy endpoints at $\alpha=0.05$ (two-sided) level in the following order:

(1) There was no difference in the proportion of subjects with a $\geq 75\%$ improvement in PASI at Week 56 between the maintenance treatment group and the withdrawal/placebo group;
(2) There was no difference in the proportion of subjects achieving PASI 100 at Week 56 between the maintenance treatment group and the withdrawal/placebo group;
(3) There was no difference in the proportion of subjects achieving static Physician Global Assessment (sPGA) clean (0) or near clean (1) at Week 56 between the maintenance treatment group and the withdrawal/placebo group;
(4) There was no difference in the proportion of subjects achieving static Physician Global Assessment (sPGA) clean (0) at Week 56 between the maintenance treatment group and the withdrawal/placebo group;
(5) There was no difference in the proportion of subjects who achieved DLQI score of 0 or 1 at Week 56 between the maintenance treatment group and the withdrawal/placebo group;
(6) Time to loss of PASI 90 between Week 32 and 56, with no difference between the maintenance treatment group and the withdrawal/placebo group (Null hypothesis: H0: HR = 1, alternative hypothesis: H1: HR1 $\neq$ 1).

The secondary efficacy endpoints of the maintenance treatment group and the withdrawal/placebo group were compared sequentially in the 100 mg and 200 mg groups. If the comparison of this endpoint between the two groups was p < 0.05, it could be considered that there was a statistically significant difference in this endpoint between the maintenance treatment group and the withdrawal/placebo group, and the comparison of the next endpoint could be performed. If a statistically significant difference is not established for this endpoint, only nominal p-values will be calculated for subsequent endpoints.

3.11.2 Statistical analysis population

This study will be summarized by treatment group based on the treatment period. The treatment period included an open-label treatment period, a randomized double-blind treatment period, and a randomized double-blind withdrawal period. The analysis populations will be defined by each phase and throughout the study (if necessary).
Intention-to-treat (ITT) set - open-label treatment period: It consists of all subjects who have received at least one dose of anti-IL-23P19 antibody prior to randomization from Week 0 to Week 20. This population was used for efficacy analyses during the open-label treatment phase.
Intention-to-treat (ITT) set - randomized double-blind treatment period: It consists of all subjects who undergo the first randomization at Week 20. The analysis of this population will be based on the treatment group to which the subjects were first randomly assigned and is used for the efficacy analysis of the randomized double - blind treatment period.

Intention-to-treat (ITT) set - randomized double-blind withdrawal responders: It consists of subjects who achieved a PASI90 response at Week 32 and were randomized for the second time. The analysis of this population will be based on the treatment group to which the subjects were randomly assigned for the second time and is used for the efficacy analysis of the randomized double - blind withdrawal period.

Per protocol set (PPS) - randomized double-blind withdrawal period: A subset of the intention - to - treat (ITT) set in the randomized double - blind withdrawal period, consisting of subjects without major protocol deviations that affect the efficacy evaluation (major protocol deviations include using concomitant medications prohibited by the protocol, not meeting the criteria for the second randomization, and incorrect administration of the study drug, etc.). The analysis of this population will be based on the treatment group that the subjects actually receive after Week 32 and is used for the supplementary analysis of efficacy indicators in the randomized double - blind withdrawal period.

Intention-to-treat (ITT) set - non-responders in the randomized, double-blind withdrawal period: It is consisted of subjects without PASI 90 response at Week 32. This population was used for efficacy analysis of non-responders after Week 32.

Intention-to-treat set (ITT) - Re-treated subjects in the randomized double-blind withdrawal period: Consisting of subjects from the intention-to-treat (ITT) set in the randomized double-blind withdrawal period who responded to the treatment initially, relapsed, and then received re-treatment. This population was used for the efficacy analysis of retreatment after relapse during the randomized, double-blind withdrawal period.

Safety set (SS) - open-label treatment period: subjects who signed the informed consent form and received at least one dose of the investigational product from Week 0 to Week 20. This population was used for safety index assessment in the open-label treatment period.

Safety set (SS) - randomized double-blind treatment period: subjects who have received at least one dose of the investigational product from Week 20 to Week 32. The analysis of this population will be based on the treatment groups that the subjects actually received from Week 20 to Week 32, and it is used for the assessment of safety indicators during the randomized double - blind treatment period.

Safety set (SS) - randomized, double-blind withdrawal period: subjects who have received at least one dose of the investigational product in Week 32 and thereafter. Analysis of this population will be based on the treatment groups that the subject actually received from Week 32 to Week 56, and will be used for the assessment of safety index during the randomized, double-blind withdrawal period.

Safety set (SS) - entire study: all subjects who have received at least one dose of the investigational product. The analysis of this population will be based on the treatment groups that the subject actually received the investigational product throughout the study for the evaluation of safety indicators throughout the study.

3.11.3 Statistical analysis method

**A. General Methods of Statistical Analysis**

Unless otherwise specified, this trial will be summarized by treatment group and by treatment period. The treatment period included an open-label treatment period (Weeks 0-20), a randomized double-blind treatment period (Week 20-32), a double-blind withdrawal period (Week 32-56), and the entire study (if necessary, evaluation of safety parameters throughout the study). The overlapping times (Week 20 and 32) will be assigned to the corresponding period based on whether the assessment/testing was performed before or after randomization.

Continuous variables will be summarized using descriptive statistics (including number of subjects, mean, standard deviation, median, Q1, Q3, minimum, and maximum), and discrete variables will be summarized using frequency and percentage.

All statistical analyses were performed using SAS v9.4 (or higher).

**B. Subject Disposition**

The number of subjects entering each treatment period, randomized in each treatment period, treated, discontinued from study treatment, and discontinued from study in this study will be statistically analyzed, respectively. The primary reasons for treatment discontinuation and/or study discontinuation will be summarized by eCRF category.

Major protocol deviations will be summarized and listed by each category.

**C. Subject Baseline Characteristics**

Demographic and baseline characteristics, diagnosis and treatment information of the study disease, medical history, and previous concomitant treatments of subjects will be summarized using descriptive statistics.

## D. Analysis of Efficacy

Efficacy endpoints will be analyzed in the corresponding intention-to-treat sets by treatment period respectively. In this trial, between-group comparisons of efficacy endpoints between the maintenance treatment group and the withdrawal/placebo group will be conducted only during the double-blind withdrawal period, and descriptive statistical summaries will be performed for efficacy endpoints in the open-label treatment period and the randomized double-blind treatment period.

1 ANALYSIS OF PRIMARY ENDPOINTS

The primary endpoint of this study was a dual endpoint: the proportion of subjects in the 100 mg group and the 200 mg group who achieved a $\geq$ 90% improvement in PASI at Week 56 before the first study drug administration, compared with the withdrawal/placebo group.

• Analysis of primary endpoint measures under the main estimand

The number and percentage of subjects in each treatment group and their corresponding 95% CIs for the primary endpoint will be summarized using descriptive statistics. For binary indicators, the chi-square test will be used to compare the statistical difference in PASI90 between the maintenance treatment group and the withdrawal/ placebo group, and the p-value will be calculated. The inter-group rate differences and their 95% CIs for the proportions of subjects in the anti-IL-23P19 antibody treatment group versus those in the placebo group were calculated using the Wald method.

Handling of intercurrent events: A therapeutic strategy will be adopted for early treatment discontinuation, and data collected subsequently will continue to be used. A composite variable strategy will be applied for the use of prohibited medications that affect efficacy evaluation; the use of other psoriasis treatments will be regarded as non-effective. After Week 32, if disease relapse occurs regardless of whether retreatment is administered and/or the subject withdraws from the study, a composite variable strategy will be adopted, and relapse will be considered be ineffective.

Imputation for missing values: imputation by non-response (NRI).

Sensitivity analysis: The missing values due to early treatment discontinuation were imputed using the predicted values in the MMRM model of the corresponding continuous variables and the LOCF method, and the missing categorical variables due to the use of other psoriasis treatments or recurrence were imputed using the NRI method. The statistical analysis method was the same as the main analysis method.

Supplementary analysis of the primary endpoint:

Different analysis populations: The primary endpoint in the PPS will be analyzed using the same method as the primary estimand described above.

Different handling strategies for intercurrent events: The while-on-treatment strategy will be used for early treatment discontinuation, only efficacy data before early treatment discontinuation will be used, and categorical variables after treatment discontinuation will be imputed using the predicted values in the MMRM model of the corresponding continuous variables. A composite variable strategy was used for other psoriasis treatments or recurrence, which were considered to have no effect.

2 Analysis of Secondary Efficacy Endpoints

• Binary variable analysis

The secondary efficacy endpoints for binary variables included the proportion of subjects with PASI75, PASI100, sPGA0 or sPGA 0/1 score, and DLQI 0/1 score at Week 56, which were analyzed in the same way as the primary endpoint analysis.

• Analysis of time to event variables

The time to event variable is the time to loss of PASI 90 response during the randomized double-blind withdrawal period, defined as the time from randomization at Week 32 to loss of PASI 90 response.

Statistical Analysis of the Primary Estimand:

The number and percentage of subjects who lost PASI90 and were censored were summarized by treatment group. The median time to loss of PASI 90 and its 95% CI will be estimated using the Kaplan-Meier method, and the KM curve will be plotted. The log-rank test will be used for inter-group comparison to calculate the p value, and the Cox model will be used to calculate the inter-group HR and its 95% CI.

Handling of intercurrent events: A therapeutic strategy will be adopted for early treatment discontinuation, and data collected subsequently will continue to be used. A composite variable strategy will be adopted for other psoriasis treatments, and PASI90 events were considered lost if other psoriasis treatments were used. Relapse after Week 32, regardless of retreatment and/or withdrawal from the study, was considered a loss of PASI 90 event using a composite variable strategy.

If an event was not observed, it was censored at the last efficacy evaluation, and no missing value was imputed.

Supplementary analysis: The statistical analysis method for loss of PASI90 in the PPS was the same as that for the primary estimand.

## 3 ANALYSIS OF OTHER EFFICACY ENDPOINTS

Other efficacy endpoints included change from baseline in DLQI score at each time point, proportion of subjects with PASI90, PASI75, PASI100, sPGA 0 score, sPGA 0/1 score, and DLQI 0/1 score at each time point, and change from baseline in each specific score of specific psoriasis.

Other efficacy endpoints described above will be summarized by treatment period using descriptive statistics: Continuous variables will be summarized using descriptive statistics, including number of subjects, mean, standard deviation, median, Q1, Q3, minimum, and maximum; frequency and percentage for categorical variables will be summarized.

MMRM will also be used for the change from baseline in DLQI of responders in the randomized, double-blind withdrawal period. In the model, treatment group, visit, and interaction between visit and treatment group will be used as fixed effects, intra-subject error as a random effect, and baseline value and its interaction with visit as covariates. The mean and its 95% CI of the change from baseline in DLQI of the maintenance treatment group and withdrawal/placebo group will be calculated, and the inter-group difference and its 95% CI will be provided, as well as the p-value for inter-group comparison.

## E. Safety Analysis

Safety endpoints were analyzed by treatment period in the corresponding safety analysis set. Overall safety throughout the trial was also analyzed in the Safety Analysis Set - Throughout the trial.

### 1 Drug exposure

The drug exposure, administration time, compliance, etc. of the subjects during each treatment period and the whole study period will be summarized.

### 2 ADVERSE EVENTS

Adverse events (AE) were coded using the Medical Dictionary for Regulatory Activities (MedDRA). The number and percentage of subjects with various types of AEs (including treatment-emergent adverse events, drug-related adverse events, AESIs, SAEs, etc.) will be summarized. The occurrence of various types of AEs will be further summarized by MedDRA system organ class and preferred term. The relationship between various types of AEs and the investigational drug and their severity will also be analyzed.

### 3 Laboratory tests

For hematology, blood biochemistry, and other parameters, the number of subjects, mean, standard deviation, median, Q1, Q3, minimum, and maximum will be descriptively summarized for each visit and their changes from baseline by group, and normal and abnormal changes before and after treatment will be summarized using a cross-classification table.

For urinalysis, cross-classification tables will be used to describe normal and abnormal changes before and after treatment.

### 4 12-lead ECG

The measured values and changes from baseline of quantitative ECG parameters will be summarized using descriptive statistics. A cross-classification table will be used to summarize the normal and abnormal changes before and after the treatment with the investigational drug.

5 Vital signs, physical examination, and other safety-related examinations

Vital sign measurements and changes from baseline will be summarized using descriptive statistics.
Subjects with abnormal changes from baseline in physical examination and other safety examinations will be listed.

### F. Immunogenicity

The positive rates of anti-drug antibody (ADA) and neutralizing antibody (NAb) of anti-IL-23P19 antibodies throughout the study will be summarized using descriptive statistics.

### F. Multiple Comparisons and Adjustments for Multiplicity

Primary endpoint:

In this study, dual endpoints were set. Only when both primary endpoints were significant at the same time, the maintenance treatment group was considered superior to the withdrawal/placebo group. No adjustment of $\alpha$ will be made.

(1) Proportion of Subjects Achieving PASI 90 at Week 56 in the 100 mg and 200 mg Maintenance Treatment and Withdrawal/Placebo Groups, respectively

When both primary and secondary endpoints achieved positive results, the secondary endpoints were sequentially tested in the following order at $\alpha$ = 0.05 (two-sided) level to verify the superiority of the anti-IL-23P19 antibody group to the placebo group. The specific sequence is as follows:

Secondary endpoints:

(2) Proportion of subjects achieving PASI75 at Week 56
(3) Proportion of subjects achieving PASI100 at Week 56
(4) Proportion of subjects achieving a static Physician Global Score (PGA) of clean (0) or near clean (1) at Week 56
(5) Proportion of subjects achieving static Physician Global Assessment (PGA) clean (0) at Week 56
(6) Proportion of subjects achieving DLQI score of 0 or 1 at Week 56
(7) Time to loss of PASI90 between Week 32 and 56.

The secondary efficacy endpoints of the maintenance treatment group and the withdrawal/placebo group were compared sequentially in the 100 mg and 200 mg groups. If the comparison of this endpoint between the two groups was p < 0.05, it could be considered that there was a statistically significant difference in this endpoint between the maintenance treatment group and the withdrawal/placebo group, and the comparison of the next endpoint could be performed. If a statistically significant difference is not established for this endpoint, only nominal p-values will be calculated for subsequent endpoints.

### 3.12 Results

The above contents show that the anti-IL23p19 antibody of the present disclosure can effectively treat moderate to severe psoriasis, has excellent efficacy and safety, and maintain treatment effect after the randomized withdrawal of the product, which has better efficacy benefits than the withdrawal of the control.
In addition, this product has the characteristics of long half-life and long interval administration, and is administered once every 12 weeks after stable treatment, so that it has the potential to maintain efficacy for a longer period of time after drug withdrawal, and more patients maintain efficacy in the same period of time after drug withdrawal, compared to the control.
Specifically, the anti-IL23P19 antibody of the present disclosure has good safety: in single-dose studies in healthy subjects and multiple-dose studies in psoriasis subjects, the overall safety of the subjects was good, the incidence of adverse events was not significantly increased compared with that of the placebo group, and no dose-dependent safety events were observed. According to the blinded data, more than 70% of the psoriasis population is expected to achieve PASI 90 at Week 16 by subcutaneous injection of 200 mg of anti-IL23P19 antibody at Week 0, 4, and 8. With maintenance doses of 100 mg and 200 mg Q12W, the long-term (Week 52) PASI90 achievement rate is expected to reach more than 85%. The two long-term maintenance dosing regimens are expected to provide an optimal convenient treatment regimen and a stable efficacy treatment regimen for the psoriasis population. Meanwhile, the method for treating moderate to severe psoriasis with the anti-IL-23p19 antibody of the present disclosure has the characteristic of long-interval administration, and is administered once every 12 weeks after stable treatment, and is expected to have the potential to maintain the efficacy for a longer time after drug withdrawal.

Specifically, the proportion of subjects who still achieved PASI 90 in different groups after Week 32 of this study was blindly estimated. Due to blinding, the 100 mg and 200 mg dose groups were not distinguished at the end of Week 32. The data are as follows:

| | Week20 | Week 32 | Withdrawal period | Week 36 | Week 40 |
|---|---|---|---|---|---|
| PASI 90 ratio | 83.3% | 89.1% | Maintenance group (anti-IL23P19 antibody) | 94.0% | 95.7% |
| | | | Withdrawal group (placebo) | 89.6% | 81.8% |

The data show that among the subjects who responded at Week 32, 94.0% and 95.7% of the subjects responded at Week 36 and Week 40, respectively, when the subjects continued the maintenance treatment with the anti-IL23P19 antibody; while among the subjects who responded at Week 32, 89.6% and 81.8% of the subjects still responded at Week 36 (14 weeks apart from the last administration at Week 20) and Week 40, respectively, when the subjects withdrew the drug and received the placebo treatment.

The exemplary embodiments of the present disclosure have been described above, and it should be understood by those skilled in the art that these disclosures are merely exemplary and that various other substitutions, adaptations, and modifications can be made within the scope of the present disclosure. Accordingly, the present disclosure is not limited to the specific embodiments set forth herein.

## Claims

1. A method for treating moderate to severe psoriasis in a subject, comprising administering to a subject in need thereof an effective amount of an anti-IL-23p19 antibody, wherein the anti-IL23p19 antibody comprises a heavy chain variable region and a light chain variable region, wherein the CDR1 of the heavy chain variable region comprises the amino acid sequence of SEQ ID NO: 1, the CDR2 of the heavy chain variable region comprises the amino acid sequence of SEQ ID NO: 2, and the CDR3 of the heavy chain variable region comprises the amino acid sequence of SEQ ID NO: 3; the CDR1 of the light chain variable region of the antibody comprises the amino acid sequence of SEQ ID NO: 4, the CDR2 of the light chain variable region comprises the amino acid sequence of SEQ ID NO: 5, and the CDR3 of the light chain variable region comprises the amino acid sequence of SEQ ID NO: 6.

2. The method according to claim 1, wherein the anti-IL23p19 antibody comprises a heavy chain variable region and a light chain variable region, wherein the heavy chain variable region comprises the sequence of SEQ ID NO: 7 or a sequence having at least 90% identity to SEQ ID NO: 7, and the light chain variable region comprises the sequence of SEQ ID NO: 8 or a sequence having at least 90% identity to SEQ ID NO: 8.

3. The method according to claim 1 or 2, wherein the anti-IL23p19 antibody comprises a heavy chain and a light chain, wherein the heavy chain comprises a sequence set forth in SEQ ID NO: 9 or comprises a sequence having at least 90% identity to SEQ ID NO: 9, and the light chain comprises a sequence set forth in SEQ ID NO: 10 or comprises a sequence having at least 90% identity to SEQ ID NO: 10.

4. The method according to any one of claims 1-3, wherein each administration dose of the anti-IL-23P19 antibody is 50 mg-500 mg, optionally, each administration dose is the same or different during the administration regimen.

5. The method according to any one of claims 1-4, wherein the method comprises administering to a subject in need thereof 200 mg of the anti-IL-23p19 antibody at Week 0, 4, and 8, administering 50-300 mg of the anti-IL-23p19 antibody at Week 20, then suspending administration of the anti-IL-23p19 antibody or continuing administration of 50-300 mg of the anti-IL-23p19 antibody at Week 32 and 44, preferably administering 50-250 mg of the anti-IL-23p19 antibody at Week 20, 32, and 44; more preferably, administering 100 mg-200 mg of the anti-IL-23p19 antibody at Week 20, 32, 44, more preferably, administrating 100 mg or 200 mg of the anti-IL-23p19 antibody at Week 20, 32, and 44.

6. The method according to any one of claims 1-5, wherein the anti-IL-23p19 antibody is administered to a subject in need thereof according to the following regimen:

> 1) administering 200 mg of the anti-IL-23p19 antibody at Week 0, 4, and 8, and administering 200 mg of the anti-IL-23p19 antibody at Week 20; or
> 2) administering 200 mg of the anti-IL-23p19 antibody at Week 0, 4, and 8, and administering 100 mg of the anti-IL-23p19 antibody at Week 20.

7. The method according to any one of claims 1-6, wherein the method further comprises testing whether the subject is responsive to treatment at Week 32.

8. The method according to any one of claims 1-7, wherein the method comprises administering to a subject who is responsive at Week 32 an anti-IL-23p19 antibody according to the following regimen:

> 1) administering 200 mg of the anti-IL-23p19 antibody at Week 0, 4, and 8, administering 100 mg of the anti-IL-23p19 antibody at Week 20, and administering 100 mg of the anti-IL-23p19 antibody at Week 32 and 44; or
> 2) administering 200 mg of the anti-IL-23p19 antibody at Week 0, 4, and 8, administering 100 mg of the anti-IL-23p19 antibody at Week 20, and then suspending administration of the anti-IL-23p19 antibody; or
> 3) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
> 4) administering 200 mg of the anti-IL-23p19 antibody at Week 0, 4, and 8, administering 200 mg of the anti-IL-23p19 antibody at Week 20, and then suspending administration of the anti-IL-23p19 antibody.

9. The method according to any one of claims 1-8, wherein for a subject who is responsive to treatment at Week 32, the method further comprises detecting whether the subject experiences relapse starting at Week 36.

10. The method according to any one of claims 1-9, wherein the method further comprises administering 200mg of anti-IL-23p19 antibody once every four weeks to subjects with relapse, starting from when relapse is confirmed until the last dose at Week 44.

11. The method according to any one of claims 1-7, wherein the method further comprises administering an anti-IL-23p19 antibody to a subject who does not respond at Week 32 according to the following regimen:

> 1) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44; or
> 2) administering 200 mg of the anti-IL-23p19 antibody at Week 0, 4, and 8, administering 100 mg of the anti-IL-23p19 antibody at Week 20, and administering 200 mg of the anti-IL-23p19 antibody at Week 32 and 44.

12. The method according to any one of claims 1-11, wherein the method further comprises:

> 1) administering 200 mg of the anti-IL-23p19 antibody at Week 0, 4, and 8, administering 200 mg of the anti-IL-23p19 antibody at Week 20, and when the subject is detected to be responsive at Week 32, administering 200 mg of the anti-IL-23p19 antibody at Week 32 and 44; or
> 2) administering 200 mg of the anti-IL-23p19 antibody at Week 0, 4, and 8, administering 200 mg of the anti-IL-23p19 antibody at Week 20, and suspending administration of the anti-IL-23p19 antibody when the subject is detected to be responsive at Week 32; or
> 3) administering 200 mg of the anti-IL-23p19 antibody at Week 0, 4, and 8, administering 200 mg of the anti-IL-23p19 antibody at Week 20, and administering 200 mg of the anti-IL-23p19 antibody at Week 32 and 44 when the subject is tested to be non-responsive at Week 32; or
> 4) administering 200 mg of the anti-IL-23p19 antibody at Week 0, 4, and 8, administering 100 mg of the anti-IL-23p19 antibody at Week 20, and when the subject is detected to be responsive at Week 32, administering 100 mg of the anti-IL-23p19 antibody at Week 32 and 44; or
> 5) administering 200 mg of the anti-IL-23p19 antibody at Week 0, 4, and 8, administering 100 mg of the anti-IL-23p19 antibody at Week 20, and suspending the administration of the anti-IL-23p19 antibody when the subject is detected to be responsive at Week 32; or
> 6) administering 200 mg of the anti-IL-23p19 antibody at Week 0, 4, and 8, administering 100 mg of the anti-IL-23p19 antibody at Week 20, and administering 200 mg of the anti-IL-23p19 antibody at Week 32 and 44 when the subject was detected to be non-responsive at Week 32.

13. The method according to claim 12, wherein for a subject detected to be responsive at Week 32, the method further comprises detecting whether the subject has relapsed starting from Week 36, wherein a subject is considered to have relapsed if the PASI response of the subject during Week 36 to Week 44 is reduced by more than 50% relative to the response at Week 32.

14. The method according to claim 13, wherein for a subject experiencing recurrence, 200 mg of the anti-IL-23p19 antibody is administered once every four Week from the time when recurrence is determined until the last dose at Week 44.

15. The method according to any one of claims 1-14, wherein the moderate to severe psoriasis is moderate to severe plaque psoriasis.

16. The method according to any one of claims 1-15, wherein the subject has moderate to severe plaque psoriasis for $\geq 6$ months, the body surface area (BSA) of the subject affected by moderate to severe plaque psoriasis is $\geq 10\%$, the psoriasis area and severity index (PASI) is $\geq 12$ points, and the static physician's global assessment score (sPGA) is $\geq 3$ points, optionally with or without psoriatic arthritis.

17. The method according to any one of claims 1-16, wherein the anti-IL-23p19 antibody is formulated into a liquid pharmaceutical composition for administration.

18. The method according to claim 17, wherein the liquid pharmaceutical composition is an injection.

19. The method according to any one of claims 1-18, wherein the anti-IL-23p19 antibody or the pharmaceutical composition is administered by subcutaneous injection.

20. Use of an anti-IL-23p19 antibody or a pharmaceutical composition comprising the anti-IL-23p19 antibody in the preparation of a medicament for treating moderate to severe plaque psoriasis, wherein the anti-IL-23p19 antibody is as defined in any one of claims 1-3.

21. The use according to claim 20, wherein the subject is as defined in claim 16.

22. The use according to claim 20 or 21, wherein the anti-IL-23pl9 antibody or the pharmaceutical composition comprising the anti-IL-23pl9 antibody is administered according to the method according to claims 1-19.

23. A single pharmaceutical dosage unit, comprising the anti-IL-23p19 antibody according to any one of claims 1-3.

24. The single pharmaceutical dosage unit according to claim 23, wherein the single pharmaceutical dosage unit comprises the following dosage of the anti-IL-23p19 antibody: 50-500 mg, preferably 50-300 mg, e.g., 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 350 mg, 400 mg, 450 mg, or 500 mg of the anti-IL-23p19 antibody.

25. The single pharmaceutical dosage unit according to claim 23 or 24, wherein the single pharmaceutical dosage unit is a single pharmaceutical dosage unit packaged in a prefilled automatic injection pen.

26. A pharmaceutical kit, comprising the single pharmaceutical dosage unit according to any one of claims 23-25, optionally, it also comprises a package insert printed with instructions for the use of an anti-IL-23p19 antibody for the preventing or treating of plaque psoriasis in a patient.

27. Use of the single pharmaceutical dosage unit according to any one of claims 23-25 or the pharmaceutical kit according to claim 26 in the manufacture of a medicament for treating moderate to severe plaque psoriasis.

28. The single pharmaceutical dosage unit according to any one of claims 23-25 or the pharmaceutical kit according to claim 26 for use in treating plaque psoriasis.

29. The use according to claim 27, or the single pharmaceutical dosage unit or the pharmaceutical kit for use according to claim 28, for use in the subject according to claim 16.

## INTERNATIONAL SEARCH REPORT

| International application No. |
|---|
| **PCT/CN2024/117492** |

**A. CLASSIFICATION OF SUBJECT MATTER**

A61K 39/395(2006.01)i; A61P17/06(2006.01)i; C07K16/24(2006.01)i; A61P37/02(2006.01)i

According to International Patent Classification (IPC) or to both national classification and IPC

**B. FIELDS SEARCHED**

Minimum documentation searched (classification system followed by classification symbols)

IPC: A61K, A61P, C07K

Documentation searched other than minimum documentation to the extent that such documents are included in the fields searched

Electronic data base consulted during the international search (name of data base and, where practicable, search terms used)

CNTXT, ENTXTC, ENTXT, OETXT, WOTXT, EPTXT, USTXT, ISI_Web of Science, CNKI, GenBank, EMBL, STN, 中国专利生物序列检索系统, China Patent Biological Sequence Search System: IL-23p19, 银屑病, 中重度, 斑块, 抗体, antibody, moderate-to-severe plaque psoriasis, SEQ ID NOs: 1-10

**C. DOCUMENTS CONSIDERED TO BE RELEVANT**

| Category* | Citation of document, with indication, where appropriate, of the relevant passages | Relevant to claim No. |
|---|---|---|
| X | WO 2020108530 A1 (INNOVENT BIOLOGICS (SUZHOU) CO., LTD.) 04 June 2020 (2020-06-04) claims 2-4, 18, 19 and 21, and description, page 8, paragraphs 5-6, page 17, paragraphs 3-7, page 19, paragraph 2 from the bottom, and pages 21-22, tables 1-3, and SEQ ID NOs: 1, 3-6, 8, 10, 12, 15 and 17 | 1-29 |
| A | CN 107206081 A (BOEHRINGER INGELHEIM INTERNATIONAL GMBH) 26 September 2017 (2017-09-26) description, embodiments 1b and 1c | 1-29 |
| A | WO 2023144699 A1 (SUN PHARMACEUTICAL INDUSTRIES LIMITED) 03 August 2023 (2023-08-03) description, embodiment 1 | 1-29 |
| A | CN 113825768 A (SUN PHARMACEUTICAL INDUSTRIES LIMITED) 21 December 2021 (2021-12-21) claims 1-44 | 1-29 |

☐ Further documents are listed in the continuation of Box C. ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **15 November 2024** | **21 November 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/117492**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

| International application No. |
| --- |
| **PCT/CN2024/117492** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **1-19**
   because they relate to subject matter not required to be searched by this Authority, namely:

   Claims 1-19 set forth a method for treating moderate-to-severe psoriasis in a subject, which belongs to subject matter for which no search is required by the International Searching Authority as defined in PCT Rule 39.1.

   The search is conducted regarding the following amended subject matter of claims 1-19 that might reasonably be expected: the use of an anti-IL-23p19 antibody in the preparation of a drug for treating moderate-to-severe psoriasis in a subject.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

EP 4 775 223 A1

## INTERNATIONAL SEARCH REPORT
### Information on patent family members

| International application No. |
|---|
| **PCT/CN2024/117492** |

| Patent document cited in search report | | | Publication date (day/month/year) | Patent family member(s) | | | Publication date (day/month/year) |
|---|---|---|---|---|---|---|---|
| WO | 2020108530 | A1 | 04 June 2020 | JP | 2022505001 | A | 14 January 2022 |
| | | | | JP | 7517999 | B2 | 17 July 2024 |
| | | | | AU | 2019386021 | A1 | 17 December 2020 |
| | | | | AU | 2019386021 | B2 | 15 February 2024 |
| | | | | KR | 20210096559 | A | 05 August 2021 |
| | | | | EP | 3904382 | A1 | 03 November 2021 |
| | | | | EP | 3904382 | A4 | 13 July 2022 |
| | | | | US | 2021115130 | A1 | 22 April 2021 |
| | | | | US | 12098195 | B2 | 24 September 2024 |
| | | | | TW | 202027791 | A | 01 August 2020 |
| | | | | TWI | 779253 | B | 01 October 2022 |
| | | | | CA | 3101670 | A1 | 04 June 2020 |
| | | | | CA | 3101670 | C | 06 February 2024 |
| | | | | AU | 2024200493 | A1 | 22 February 2024 |
| CN | 107206081 | A | 26 September 2017 | JP | 2020125338 | A | 20 August 2020 |
| | | | | JP | 7044824 | B2 | 30 March 2022 |
| | | | | JP | 2020125340 | A | 20 August 2020 |
| | | | | JP | 7042298 | B2 | 25 March 2022 |
| | | | | JP | 2022081644 | A | 31 May 2022 |
| | | | | AU | 2021286378 | A1 | 20 January 2022 |
| | | | | MX | 2017010037 | A | 15 May 2018 |
| | | | | EP | 3253794 | A1 | 13 December 2017 |
| | | | | NZ | 733136 | A | 26 April 2024 |
| | | | | IL | 307578 | A | 01 December 2023 |
| | | | | CA | 2972995 | A1 | 11 August 2016 |
| | | | | CL | 2017001960 | A1 | 20 April 2018 |
| | | | | EA | 201791734 | A1 | 31 January 2018 |
| | | | | AU | 2016215535 | A1 | 13 July 2017 |
| | | | | AU | 2016215535 | B2 | 16 September 2021 |
| | | | | US | 2016222102 | A1 | 04 August 2016 |
| | | | | JP | 2020125339 | A | 20 August 2020 |
| | | | | JP | 7042297 | B2 | 25 March 2022 |
| | | | | WO | 2016126638 | A1 | 11 August 2016 |
| | | | | IL | 253118 | A0 | 31 August 2017 |
| | | | | BR | 112017014684 | A2 | 09 January 2018 |
| | | | | SG | 11201705728 | RA | 30 August 2017 |
| | | | | MX | 2022006573 | A | 11 July 2022 |
| | | | | SG | 10202103879 | YA | 28 May 2021 |
| | | | | MX | 2022006575 | A | 11 July 2022 |
| | | | | JP | 2018505882 | A | 01 March 2018 |
| | | | | JP | 6758304 | B2 | 23 September 2020 |
| | | | | KR | 20170120616 | A | 31 October 2017 |
| | | | | PH | 12017501378 | A1 | 08 January 2018 |
| WO | 2023144699 | A1 | 03 August 2023 | AU | 2023211042 | A1 | 08 August 2024 |
| | | | | AU | 2023211042 | A9 | 15 August 2024 |
| | | | | IL | 314265 | A | 01 September 2024 |
| | | | | US | 2023303678 | A1 | 28 September 2023 |
| CN | 113825768 | A | 21 December 2021 | IL | 287213 | A | 01 December 2021 |
| | | | | AU | 2020259375 | A1 | 28 October 2021 |
| | | | | BR | 112021020612 | A2 | 28 December 2021 |

Form PCT/ISA/210 (patent family annex) (July 2022)

36

**INTERNATIONAL SEARCH REPORT**
Information on patent family members

International application No.

**PCT/CN2024/117492**

| Patent document cited in search report | Publication date (day/month/year) | Patent family member(s) | | | Publication date (day/month/year) |
|---|---|---|---|---|---|
| | | US | 2022298233 | A1 | 22 September 2022 |
| | | CA | 3143604 | A1 | 22 October 2020 |
| | | JOP | 20210279 | A1 | 30 January 2023 |
| | | EP | 3956357 | A1 | 23 February 2022 |
| | | EP | 3956357 | A4 | 04 January 2023 |
| | | WO | 2020212874 | A1 | 22 October 2020 |
| | | SG | 11202111056 | YA | 29 November 2021 |
| | | MA | 55729 | A | 23 February 2022 |
| | | KR | 20210140780 | A | 23 November 2021 |
| | | MX | 2021012652 | A | 24 January 2022 |
| | | JP | 2022529266 | A | 20 June 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2021093219 W

- CN 2019121261 W

**Non-patent literature cited in the description**

- **OPPMANN et al.** *Immunity*, 2000, vol. 13, 715
- **BRAVO** ; **HEATH**. *EMBO J.,* 2000, vol. 19, 2399-2411
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948

- **KABAT et al.** Sequences of Proteins of Immunological Interest. U.S. Department of Health and Human Services, 1987
- **KABAT et al.** Sequences of Proteins of Immunological Interest. National Institutes of Health, 1991